# EUROPEAN PATENT SPECIFICATION

(11) **EP 1 513 923 B1**
(45) Date of publication and mention of the grant of the patent: **18.07.2007**
(21) Application number: 03731489.5
(22) Date of filing: 30.05.2003
(51) Int. Cl.: C12N 1/14, C12N 1/16, C12P 21/04, C12P 23/00

(54) **METHODS AND MATERIALS FOR THE PRODUCTION OF D-LACTIC ACID IN YEAST**
VERFAHREN UND MATERIALIEN ZUR PRODUKTION VON D-MILCHSÄURE IN HEFE
PROCEDES ET MATERIAUX DESTINES A PRODUIRE DE L'ACIDE D-LACTIQUE DANS UNE LEVURE

(30) Priority: 30.05.2002 US 384333 P
(43) Date of publication of application: 16.03.2005
(73) Proprietor: NatureWorks LLC, Minnetonka MN 55345 (US)
(72) Inventor: RAJGARHIA, Vineet, Minnetonka, MN 55343 (US); DUNDON, Catherine, Asleson, Minneapolis, MN 55406 (US); OLSON, Stacey, St. Bonifacius, MN 55375 (US); SUOMINEN, Pirkko, Maple Grove, MN 55311 (US); HAUSE, Ben, Jordan, MN 55352 (US)
(74) Representative: Portal, Frédéric
(86) International application number: PCT/US2003/017310
(87) International publication number: WO 2003/102201

(56) References cited:
- JP-A- 2002 136 293
- US-B1- 6 485 947
- DEQUIN S ET AL: "Mixed lactic acid-alcoholic fermentation by Saccharomyces cerevisiae expressing the Lactobacillus casei L(+)-LDH." BIO/TECHNOLOGY (NATURE PUBLISHING COMPANY) FEB 1994, vol. 12, no. 2, February 1994 (1994-02), pages 173-177, XP009063893 ISSN: 0733-222X
- CHELSTOWSKA A ET AL: "Signalling between mitochondria and the nucleus regulates the expression of a new D-lactate dehydrogenase activity in yeast." YEAST (CHICHESTER, ENGLAND) 30 SEP 1999, vol. 15, no. 13, 30 September 1999 (1999-09-30), pages 1377-1391, XP009039927 ISSN: 0749-503X

### SEQUENCE LISTING

<110> Cargill Dow Polymers LLC
   Rajgarhia, Vineet
   Asleson, Catherine
   olson, Stacey
   Suominen, Pirkko
<120> Methods and Materials for the Production of D-Lactic Acid in Yeast
<130> 00-1237-Q
<150> US 60/384,333
   <151> 2002-05-30
<160> 43
<170> PatentIn version 3.2
<210> 1
   <211> 848
   <212> DNA
   <213> Saccaromyces cerevisiae;
<400> 1
   gcggccgcgg atcgctcttc cgctatcgat taattttttt ttctttcctc tttttattaa      60
   ccttaatttt tattttagat tcctgacttc aactcaagac gcacagatat tataacatct     120
   gcacaatagg catttgcaag aattactcgt gagtaaggaa agagtgagga actatcgcat     180
   acctgcattt aaagatgccg atttgggcgc gaatccttta ttttggcttc accctcatac     240
   tattatcagg gccagaaaaa ggaagtgttt ccctccttct tgaattgatg ttaccctcat     300
   aaagcacgtg gcctcttatc gagaaagaaa ttaccgtcgc tcgtgatttg tttgcaaaaa     360
   gaacaaaact gaaaaaaccc agacacgctc gacttcctgt cttcctattg attgcagctt     420
   ccaatttcgt cacacaacaa ggtcctagcg acggctcaca ggttttgtaa caagcaatcg     480
   aaggttctgg aatggcggga aagggtttag taccacatgc tatgatgccc actgtgatct     540
   ccagagcaaa gttcgttcga tcgtactgtt actctctctc tttcaaacag aattgtccga     600
   atcgtgtgac aacaacagcc tgttctcaca cactcttttc ttctaaccaa gggggtggtt     660
   tagtttagta gaacctcgtg aaacttacat ttacatatat ataaacttgc ataaattggt     720
   caatgcaaga aatacatatt tggtcttttc taattcgtag tttttcaagt tcttagatgc     780
   tttctttttc tcttttttac agatcatcaa ggaagtaatt atctactttt tacaacaaat     840
   ctagaatt                                                              848
<210> 2
   <211> 376
   <212> DNA
   <213> Saccaromy cescerevisiae;
<400> 2
   gtagatacat tgatgctatc aatccagaga actggaaaga ttgtgtagcc ttgaaaaacg 60
   gtgaaactta cgggtccaag attgtctaca gattttcctg atttgccagc ttactatcct 120
   tcttgaaaat atgcactcta tatcttttag ttcttaattg caacacatag atttgctgta 180
   taacgaattt tatgctattt tttaaatttg gagttcagtg ataaaagtgt cacagcgaat 240
   ttcctcacat gtagggaccg aattgtttac aagttctctg taccaccatg gagacatcaa 300
   aaattgaaaa tctatggaaa gatatggacg gtagcaacaa gaatatagca cgagccgcgg 360
   atttatttcg ttacgc 376
<210> 3
   <211> 27
   <212> DNA
   <213> Artificial Sequence;
<220>
   <223> PSPDCS1 primer
<400> 3
   ccatcgataa caagctcatg caaagag 27
<210> 4
   <211> 28
   <212> DNA
   <213> Artificial sequence;
<220>
   <223> PSPDCAS2 primer
<400> 4
   gctctagatt tgactgtgtt attttgcg 28
<210> 5
   <211> 21
   <212> DNA
   <213> Artificial sequence;
<220>
   <223> BM1270 primer
<400> 5
   cctgagtcca cgtcattatt c 21
<210> 6
   <211> 22
   <212> DNA
   <213> Artificial sequence;
<220>
   <223> BM179 primer
<400> 6
   tgaagctatt tattcttgtt ac 22
<210> 7
   <211> 27
   <212> DNA
   <213> Artificial sequence;
<220>
   <223> Bmeg5' primer
<400> 7
   gctctagatg aaaacacaat ttacacc 27
<210> 8
   <211> 28
   <212> DNA
   <213> Artificial sequence;
<220>
   <223> Bmeg3' primer
<400> 8
   atggatcctt acacaaaagc tctgtcgc 28
<210> 9
   <211> 30
   <212> DNA
   <213> Artificial sequence;
<220>
   <223> 5' G fragment primer
<400> 9
   gctctagatg agccatattc aacgggaaac 30
<210> 10
   <211> 30
   <212> DNA
   <213> Artificial sequence;
<220>
   <223> 3' G fragment primer
<400> 10
   atggatcctt agaaaaactc atcgagcatc 30
<210> 11
   <211> 24
   <212> DNA
   <213> Artificial sequence;
<220>
   <223> SO-M2 primer
<400> 11
   cttccagtcc agcaggtcga ccag 24
<210> 12
   <211> 18
   <212> DNA
   <213> Artificial sequence;
<220>
   <223> SO-M1 primer
<400> 12
   gtccagcatg tcgaccag 18
<210> 13
   <211> 20
   <212> DNA
   <213> Artificial Sequence;
<220>
   <223> SO-M4 primer
<400> 13
   gaacgaaacg aacttctctc 20
<210> 14
   <211> 20
   <212> DNA
   <213> Artificial sequence;
<220>
   <223> SO-M5 primer
<400> 14
   cttggaactt cttgtcagtg 20
<210> 15
   <211> 21
   <212> DNA
   <213> Artificial sequence;
<220>
   <223> SO4549 primer
<400> 15
   ccatccgaag aagtcgatct c 21
<210> 16
   <211> 21
   <212> DNA
   <213> Artificial Sequence;
<220>
   <223> SO285 primer
<400> 16
   cttgccatcc tatggaactg c 21
<210> 17
   <211> 20
   <212> DNA
   <213> Artificial sequence;
<220>
   <223> -SO2740 primer
<400> 17
   gaaggctggg aattgagtga 20
<210> 18
   <211> 20
   <212> DNA
   <213> Artificial sequence;
<220>
   <223> SO2444 primer
<400> 18
   gctccatctg aaatcgacag 20
<210> 19
   <211> 29
   <212> DNA
   <213> Artificial sequence;
<220>
   <223> 5'HYGXBA1 primer
<400> 19
   aagctctaga tgaaaaagcc tgaactcac 29
<210> 20
   <211> 29
   <212> DNA
   <213> Artificial sequence;
<220>
   <223> 3'HYGBAMH1 primer
<400> 20
   cgcggatccc tattcctttg ccctcggac 29
<210> 21
   <211> 28
   <212> DNA
   <213> Artificial Sequence;
<220>
   <223> VR150 primer
<400> 21
   ggttggattt atgacaaagg ttttgctt 28
<210> 22
   <211> 30
   <212> DNA
   <213> Artificial Sequence;
<220>
   <223> VR153 primer
<400> 22
   aattaaaact tgttcttgtt caaagcaact 30
<210> 23
   <211> 28
   <212> DNA
   <213> Artificial sequence;
<220>
   <223> VR165 primer
<400> 23
   cgtctagatt tatgacaaag gttttgct 28
<210> 24
   <211> 29
   <212> DNA
   <213> Artificial sequence;
<220>
   <223> VR166 primer
<400> 24
   gcggatcctt aaaacttgtt cttgttcaa 29
<210> 25
   <211> 20
   <212> DNA
   <213> Artificial Sequence;
<220>
   <223> VR161 primer
<400> 25
   agttggtgta tttaacaagg 20
<210> 26
   <211> 27
   <212> DNA
   <213> Artificial sequence;
<220>
   <223> VR142 primer
<400> 26
   gtgacaccct gtgcacggcg ggagatg 27
<210> 27
   <211> 20
   <212> DNA
   <213> Artificial sequence;
<220>
   <223> VR173 primer
<400> 27
   gcgacggctc acaggttttg 20
<210> 28
   <211> 27
   <212> DNA
   <213> Artificial Sequence;
<220>
   <223> VR170 primer
<400> 28
   cttgtcttga cgtaatacac gtgcagc 27
<210> 29
   <211> 20
   <212> DNA
   <213> Artificial Sequence;
<220>
   <223> oCA85 primer
<400> 29
   ggaccgatgg ctgtgtagaa 20
<210> 30
   <211> 20
   <212> DNA
   <213> Artificial Sequence;
<220>
   <223> oCA80 primer
<400> 30
   tcgcttacct cggtacagaa 20
<210> 31
   <211> 30
   <212> DNA
   <213> Artificial Sequence;
<220>
   **<223> 5'-Flank 5' primer**
<400> 31
   caagaaggta cccctctcta aacttgaaca 30
<210> 32
   <211> 32
   <212> DNA
   <213> Artificial sequence;
<220>
   **<223> 5'-Flank 3' primer**
<400> 32
   gtaattcctg caggtgcaat tatttggttt gg 32
<210> 33
   <211> 32
   <212> DNA
   <213> Artificial sequence;
<220>
   <223> 3'-Flank 5' primer
<400> 33
   ccaagccctg caggagaggg agaggataaa ga 32
<210> 34
   <211> 30
   <212> DNA
   <213> Artificial sequence;
<220>
   <223> 3'-Flank 3' primer
<400> 34
   ctcgtaacgc gtgtacaagt tgtggaacaa 30
<210> 35
   <211> 32
   <212> DNA
   <213> Artificial sequence;
<220>
   <223> Lh-D-LDH 5' primer
<400> 35
   tttttacctg caggctagat ttatgacaaa gg 32
<210> 36
   <211> 32
   <212> DNA
   <213> Artificial sequence;
<220>
   <223> Lh-D-LDH 3' primer
<400> 36
   tctacccctg caggaaaact tgttcttgtt ca 32
<210> 37
   <211> 21
   <212> DNA
   <213> Artificial sequence;
<220>
   <223> PDC1 Chromosome 5' primer
<400> 37
   aagcaccaag gccttcaaca g 21
<210> 38
   <211> 27
   <212> DNA
   <213> Artificial sequence;
<220>
   <223> D-LDH 3' primer
<400> 38
   cttgtcttga cgtaatacac gtgcagc 27
**<210> 39**
   **<211> 21**
   <212> DNA
   <213> Artificial sequence;
<220>
   <223> PDC1 5' primer
<400> 39
   cgcaaagaaa agctccacac c 21
<210> 40
   <211> 21
   <212> DNA
   <213> Artificial sequence;
<220>
   <223> PDC1 3' primer
<400> 40
   cccatacgct tataatcccc c 21
<210> 41
   <211> 1235
   <212> DNA
   <213> saccaromyces cerevisiae;
<400> 41
   ggccgcggat cgctcttccg ctatcgatta attttttttt ctttcctctt tttattaacc 60
   ttaattttta ttttagattc ctgacttcaa ctcaagacgc acagatatta taacatctgc 120
   acaataggca tttgcaagaa ttactcgtga gtaaggaaag agtgaggaac tatcgcatac   180
   ctgcatttaa agatgccgat ttgggcgcga atcctttatt ttggcttcac cctcatacta   240
   ttatcagggc cagaaaaagg aagtgtttcc ctccttcttg aattgatgtt accctcataa   300
   agcacgtggc ctcttatcga gaaagaaatt accgtcgctc gtgatttgtt tgcaaaaaga   360
   acaaaactga aaaaacccag acacgctcga cttcctgtct tcctattgat tgcagcttcc   420
   aatttcgtca cacaacaagg tcctagcgac ggctcacagg ttttgtaaca agcaatcgaa   480
   ggttctggaa tggcgggaaa gggtttagta ccacatgcta tgatgcccac tgtgatctcc   540
   agagcaaagt tcgttcgatc gtactgttac tctctctctt tcaaacagaa ttgtccgaat   600
   cgtgtgacaa caacagcctg ttctcacaca ctcttttctt ctaaccaagg gggtggttta   660
   gtttagtaga acctcgtgaa acttacattt acatatatat aaacttgcat aaattggtca   720
   atgcaagaaa tacatatttg gtcttttcta attcgtagtt tttcaagttc ttagatgctt   780
   tctttttctc ttttttacag atcatcaagg aagtaattat ctacttttta caacaaatct   840
   agaattcgga tccggtagat acattgatgc tatcaatcaa gagaactgga aagattgtgt   900
   aaccttgaaa aacggtgaaa cttacgggtc caagaccctc tacagatttt cctgatttgc   960
   cagcttacta tccttcttga aaatatgcac tctatatctt ttagttctta attgcaacac  1020
   atagatttgc tgtataacga attttatgct attttttaaa tttggagttc agtgataaaa  1080
   gtgtcacagc gaatttcctc acatgtagga ccgaattgtt tacaagttct ctgtaccacc  1140
   atggagacat caaagattga aaatctatgg aaagatatgg acggtagcaa caagaatata  1200
   gcacgagccg cggatttatt tcgttacgca tgcgc                              1235
<210> 42
   <211> 1314
   <212> DNA
   <213> Saccaromyces cerevisiae;
<400> 42
   ggccgcggat cgctcttccg ctatcgataa caagctcatg caaagaggtg gtacccgcac     60
   gccgaaatgc atgcaagtaa cctattcaaa gtaatatctc atacatgttt catgagggta    120
   acaacatgcg actgggtgag catatgttcc gctgatgtga tgtgcaagat aaacaagcaa    180
   ggcagaaact aacttcttct tcatgtaata aacacacccc gcgtttattt acctatctct    240
   aaacttcaac accttatatc ataactaata tttcttgaga taagcacact gcacccatac    300
   cttccttaaa aacgtagctt ccagtttttg gtggttccgg cttccttccc gattccgccc    360
   gctaaacgca tatttttgtt gcctggtggc atttgcaaaa tgcataacct atgcatttaa    420
   aagattatgt atgctcttct gacttttcgt gtgatgaggc tcgtggaaaa aatgaataat    480
   ttatgaattt gagaacaatt ttgtgttgtt acggtatttt actatggaat aatcaatcaa    540
   ttgaggattt tatgcaaata tcgtttgaat atttttccga ccctttgagt acttttcttc    600
   ataattgcat aatattgtcc gctgcccctt tttctgttag acggtgtctt gatctacttg    660
   ctatcgttca acaccacctt attttctaac tatttttttt ttagctcatt tgaatcagct    720
   tatggtgatg gcacattttt gcataaacct agctgtcctc gttgaacata ggaaaaaaaa    780
   atatataaac aaggctcttt cactctcctt gcaatcagat ttgggtttgt tccctttatt    840
   ttcatatttc ttgtcatatt cctttctcaa ttattatttt ctactcataa cctcacgcaa    900
   aataacacag tcaaatctag aattcggatc cggtagatac attgatgcta tcaatccaga    960
   gaactggaaa gattgtgtag ccttgaaaaa cggtgaaact tacgggtcca agattgtcta   1020
   cagattttcc tgatttgcca gcttactatc cttcttgaaa atatgcactc tatatctttt   1080
   agttcttaat tgcaacacat agatttgctg tataacgaat tttatgctat tttttaaatt   1140
   tggagttcag tgataaaagt gtcacagcga atttcctcac atgtagggac cgaattgttt   1200
   acaagttctc tgtaccacca tggagacatc aaaaattgaa aatctatgga aagatatgga   1260
   cggtagcaac aagaatatag cacgagccgc ggatttattt cgttacgcat gcgc         1314
<210> 43
   <211> 1014
   <212> DNA
   <213> Lactobacillus helveticus;
<400> 43
   atgacaaagg tttttgctta cgctattcga aaagacgaag aaccattctt gaatgaatgg      60
   aaggaagctc acaaggatat cgatgttgat tacactgata aacttttgac tcctgaaact     120
   gctaagctag ctaagggtgc tgacggtgtt gttgtttacc aacaattaga ctacactgca     180
   gatactcttc aagctttagc agacgctggc gtaactaaga tgtcattacg taacgttggt     240
   gttgacaaca ttgatatgga caaggctaag gaattaggtt tccaaattac caatgttcct     300
   gtttactcac caaacgctat tgctgaacat gctgctattc aggctgcacg tgtattacgt     360
   caagacaagc gcatggacga aaagatggct aaacgtgact tacgttgggc accaactatc     420
   ggccgtgaag ttcgtgacca agttgtcggt gttgttggta ctggtcacat tggtcaagta     480
   tttatgcgta ttatggaagg tttcggtgca aaggttattg cttacgatat cttcaagaac     540
   ccagaacttg aaaagaaggg ttactacgtt gactcacttg acgacttgta caagcaagct     600
   gatgtaattt cacttcacgt accagatgtt ccagctaacg ttcacatgat caacgacaag     660
   tcaatcgctg aaatgaaaga cggcgttgta attgtaaact gctcacgtgg tcgacttgtt     720
   gacactgacg ctgtaatccg tggtttggac tcaggcaaga tcttcggctt cgttatggat     780
   acttacgaag acgaagttgg tgtatttaac aaggattggg aaggtaaaga attcccagac     840
   aagcgtttgg cagacttaat tgatcgtcca aacgtattgg taactccaca caccgccttc     900
   tacactactc acgctgtacg taacatggtt gttaaggcat tcaacaacaa cttgaagtta     960
   atcaacggcg aaaagccaga ttctccagtt gctttgaaca agaacaagtt ttaa          1014

### SEQUENCE LISTING

<110> Cargill Dow LLC
<120> Methods and Materials for the Production of D-Lactic Acid in Yeast
<130> 3J336220 004 EP ECT GPO/HW/SG
<150> us 60/384,333
   <151> 2002-05-30
<160> 43
<170> PatentIn version 3.2
<210> 1
   <211> 848
   <212> DNA
   <213> Saccaromyces cerevisiae;
<400> 1
   gcggccgcgg atcgctcttc cgctatcgat taattttttt ttctttcctc tttttattaa     60
   ccttaatttt tattttagat tcctgacttc aactcaagac gcacagatat tataacatct    120
   gcacaatagg catttgcaag aattactcgt gagtaaggaa agagtgagga actatcgcat    180
   acctgcattt aaagatgccg atttgggcgc gaatccttta ttttggcttc accctcatac    240
   tattatcagg gccagaaaaa ggaagtgttt ccctccttct tgaattgatg ttaccctcat    300
   aaagcacgtg gcctcttatc gagaaagaaa ttaccgtcgc tcgtgatttg tttgcaaaaa    360
   gaacaaaact gaaaaaaccc agacacgctc gacttcctgt cttcctattg attgcagctt    420
   ccaatttcgt cacacaacaa ggtcctagcg acggctcaca ggttttgtaa caagcaatcg    480
   aaggttctgg aatggcggga aagggtttag taccacatgc tatgatgccc actgtgatct    540
   ccagagcaaa gttcgttcga tcgtactgtt actctctctc tttcaaacag aattgtccga    600
   atcgtgtgac aacaacagcc tgttctcaca cactcttttc ttctaaccaa gggggtggtt    660
   tagtttagta gaacctcgtg aaacttacat ttacatatat ataaacttgc ataaattggt    720
   caatgcaaga aatacatatt tggtcttttc taattcgtag tttttcaagt tcttagatgc    780
   tttctttttc tcttttttac agatcatcaa ggaagtaatt atctactttt tacaacaaat    840
   ctagaatt                                                             848
<210> 2
   <211> 376
   <212> DNA
   <213> Saccaromy cescerevisiae;
<400> 2
   gtagatacat tgatgctatc aatccagaga actggaaaga ttgtgtagcc ttgaaaaacg     60
   gtgaaactta cgggtccaag attgtctaca gattttcctg atttgccagc ttactatcct    120
   tcttgaaaat atgcactcta tatcttttag ttcttaattg caacacatag atttgctgta    180
   taacgaattt tatgctattt tttaaatttg gagttcagtg ataaaagtgt cacagcgaat    240
   ttcctcacat gtagggaccg aattgtttac aagttctctg taccaccatg gagacatcaa    300
   aaattgaaaa tctatggaaa gatatggacg gtagcaacaa gaatatagca cgagccgcgg    360
   atttatttcg ttacgc                                                    376
<210> 3
   <211> 27
   <212> DNA
   <213> Artificial sequence;
<220>
   <223> PSPDCS1 primer
<400> 3
   ccatcgataa caagctcatg caaagag 27
<210> 4
   <211> 28
   <212> DNA
   <213> Artificial Sequence;
<220>
   <223> PSPDCAS2 primer
<400> 4
   gctctagatt tgactgtgtt attttgcg 28
<210> 5
   <211> 21
   <212> DNA
   <213> Artificial Sequence;
<220>
   <223> BM1270 primer
<400> 5
   cctgagtcca cgtcattatt c 21
<210> 6
   <211> 22
   <212> DNA
   <213> Artificial Sequence;
<220>
   <223> BM179 primer
<400> 6
   tgaagctatt tattcttgtt ac 22
<210> 7
   <211> 27
   <212> DNA
   <213> Artificial Sequence;
<220>
   <223> Bmeg5' primer
<400> 7
   gctctagatg aaaacacaat ttacacc 27
<210> 8
   <211> 28
   <212> DNA
   <213> Artificial sequence;
<220>
   <223> Bmeg3' primer
<400> 8
   atggatcctt acacaaaagc tctgtcgc 28
<210> 9
   <211> 30
   <212> DNA
   <213> Artificial sequence;
<220>
   <223> 5' G fragment primer
<400> 9
   gctctagatg agccatattc aacgggaaac 30
<210> 10
   <211> 30
   <212> DNA
   <213> Artificial sequence;
<220>
   <223> 3' G fragment primer
<400> 10
   atggatcctt agaaaaactc atcgagcatc 30
<210> 11
   <211> 24
   <212> DNA
   <213> Artificial sequence;
<220>
   <223> SO-M2 primer
<400> 11
   cttccagtcc agcaggtcga ccag 24
<210> 12
   <211> 18
   <212> DNA
   <213> Artificial sequence;
<220>
   <223> S0-M1 primer
<400> 12
   gtccagcatg tcgaccag 18
<210> 13
   <211> 20
   <212> DNA
   <213> Artificial sequence;
<220>
   <223> SO-M4 primer
<400> 13
   gaacgaaacg aacttctctc 20
<210> 14
   <211> 20
   <212> DNA
   <213> Artificial sequence;
<220>
   <223> SO-M5 primer
<400> 14
   cttggaactt cttgtcagtg 20
<210> 15
   <211> 21
   <212> DNA
   <213> Artificial sequence;
<220>
   <223> S04S49 primer
<400> 15
   ccatccgaag aagtcgatct c 21
<210> 16
   <211> 21
   <212> DNA
   <213> Artificial sequence;
<220>
   <223> SO285 primer
<400> 16
   cttgccatcc tatggaactg c 21
<210> 17
   <211> 20
   <212> DNA
   <213> Artificial sequence;
<220>
   <223> -SO2740 primer
<400> 17
   gaaggctggg aattgagtga 20
<210> 18
   <211> 20
   <212> DNA
   <213> Artificial sequence;
<220>
   <223> SO2444 primer
<400> 18
   gctccatctg aaatcgacag 20
<210> 19
   <211> 29
   <212> DNA
   <213> Artificial sequence;
<220>
   <223> 5'HYGXBA1 primer
<400> 19
   aagctctaga tgaaaaagcc tgaactcac 29
<210> 20
   <211> 29
   <212> DNA
   <213> Artificial sequence;
<220>
   <223> 3'HYGBAMH1 primer
<400> 20
   cgcggatccc tattcctttg ccctcggac 29
<210> 21
   <211> 28
   <212> DNA
   <213> Artificial sequence;
<220>
   <223> VR150 primer
<400> 21
   ggttggattt atgacaaagg ttttgctt 28
<210> 22
   <211> 30
   <212> DNA
   <213> Artificial sequence;
<220>
   <223> VR153 primer
<400> 22
   aattaaaact tgttcttgtt caaagcaact 30
<210> 23
   <211> 28
   <212> DNA
   <213> Artificial Sequence;
<220>
   <223> VR165 primer
<400> 23
   cgtctagatt tatgacaaag gttttgct 28
<210> 24
   <211> 29
   <212> DNA
   <213> Artificial Sequence;
<220>
   <223> VR166 primer
<400> 24
   gcggatcctt aaaacttgtt cttgttcaa 29
<210> 25
   <211> 20
   <212> DNA
   <213> Artificial Sequence;
<220>
   <223> VR161 primer
<400> 25
   agttggtgta tttaacaagg 20
<210> 26
   <211> 27
   <212> DNA
   <213> Artificial sequence;
<220>
   <223> VR142 primer
<400> 26
   gtgacaccct gtgcacggcg ggagatg 27
<210> 27
   <211> 20
   <212> DNA
   <213> Artificial Sequence;
<220>
   <223> VR173 primer
<400> 27
   gcgacggctc acaggttttg 20
<210> 28
   <211> 27
   <212> DNA
   <213> Artificial sequence;
<220>
   <223> VR170 primer
<400> 28
   cttgtcttga cgtaatacac gtgcagc 27
<210> 29
   <211> 20
   <212> DNA
   <213> Artificial sequence;
<220>
   <223> oCA85 primer
<400> 29
   ggaccgatgg ctgtgtagaa 20
<210> 30
   <211> 20
   <212> DNA
   <213> Artificial Sequence;
<220>
   <223> oCA80 primer
<400> 30
   tcgcttacct cggtacagaa 20
<210> 31
   <211> 30
   <212> DNA
   <213> Artificial sequence;
<220>
   <223> 5'-Flank 5' primer
<400> 31
   caagaaggta cccctctcta aacttgaaca 30
<210> 32
   <211> 32
   <212> DNA
   <213> Artificial sequence;
<220>
   <223> 5'-Flank 3' primer
<400> 32
   gtaattcctg caggtgcaat tatttggttt gg 32
<210> 33
   <211> 32
   <212> DNA
   <213> Artificial sequence;
<220>
   <223> 3'-Flank 5' primer
<400> 33
   ccaagccctg caggagaggg agaggataaa ga 32
<210> 34
   <211> 30
   <212> DNA
   <213> Artificial Sequence;
<220>
   <223> 3'-Flank 3' primer
<400> 34
   ctcgtaacgc gtgtacaagt tgtggaacaa 30
<210> 35
   <211> 32
   <212> DNA
   <213> Artificial Sequence;
<220>
   <223> Lh-D-LDH 5' primer
<400> 35
   tttttacctg caggctagat ttatgacaaa gg 32
<210> 36
   <211> 32
   <212> DNA
   <213> Artificial sequence;
<220>
   <223> Lh-D-LDH 3' primer
<400> 36
   tctacccctg caggaaaact tgttcttgtt ca 32
<210> 37
   <211> 21
   <212> DNA
   <213> Artificial Sequence;
<220>
   <223> PDC1 Chromosome 5' primer
<400> 37
   aagcaccaag gccttcaaca g 21
<210> 38
   <211> 27
   <212> DNA
   <213> Artificial Sequence;
<220>
   <223> D-LDH 3' primer
<400> 38
   cttgtcttga cgtaatacac gtgcagc 27
<210> 39
   <211> 21
   <212> DNA
   <213> Artificial sequence;
<220>
   <223> PDC1 5' primer
<400> 39
   cgcaaagaaa agctccacac c 21
<210> 40
   <211> 21
   <212> DNA
   <213> Artificial Sequence;
<220>
   <223> PDC1 3' primer
<400> 40
   cccatacgct tataatcccc c 21
<210> 41
   <211> 1235
   <212> DNA
   <213> Saccaromyces cerevisiae;
<400> 41
   ggccgcggat cgctcttccg ctatcgatta attttttttt ctttcctctt tttattaacc     60
   ttaattttta ttttagattc ctgacttcaa ctcaagacgc acagatatta taacatctgc    120
   acaataggca tttgcaagaa ttactcgtga gtaaggaaag agtgaggaac tatcgcatac    180
   ctgcatttaa agatgccgat ttgggcgcga atcctttatt ttggcttcac cctcatacta    240
   ttatcagggc cagaaaaagg aagtgtttcc ctccttcttg aattgatgtt accctcataa    300
   agcacgtggc ctcttatcga gaaagaaatt accgtcgctc gtgatttgtt tgcaaaaaga    360
   acaaaactga aaaaacccag acacgctcga cttcctgtct tcctattgat tgcagcttcc    420
   aatttcgtca cacaacaagg tcctagcgac ggctcacagg ttttgtaaca agcaatcgaa    480
   ggttctggaa tggcgggaaa gggtttagta ccacatgcta tgatgcccac tgtgatctcc    540
   agagcaaagt tcgttcgatc gtactgttac tctctctctt tcaaacagaa ttgtccgaat    600
   cgtgtgacaa caacagcctg ttctcacaca ctcttttctt ctaaccaagg gggtggttta    660
   gtttagtaga acctcgtgaa acttacattt acatatatat aaacttgcat aaattggtca    720
   atgcaagaaa tacatatttg gtcttttcta attcgtagtt tttcaagttc ttagatgctt    780
   tctttttctc ttttttacag atcatcaagg aagtaattat ctacttttta caacaaatct     840
   agaattcgga tccggtagat acattgatgc tatcaatcaa gagaactgga aagattgtgt    900
   aaccttgaaa aacggtgaaa cttacgggtc caagaccctc tacagatttt cctgatttgc    960
   cagcttacta tccttcttga aaatatgcac tctatatctt ttagttctta attgcaacac   1020
   atagatttgc tgtataacga attttatgct attttttaaa tttggagttc agtgataaaa   1080
   gtgtcacagc gaatttcctc acatgtagga ccgaattgtt tacaagttct ctgtaccacc   1140
   atggagacat caaagattga aaatctatgg aaagatatgg acggtagcaa caagaatata   1200
   gcacgagccg cggatttatt tcgttacgca tgcgc                              1235
<210> 42
   <211> 1314
   <212> DNA
   <213> Saccaromyces cerevisiae;
<400> 42
   ggccgcggat cgctcttccg ctatcgataa caagctcatg caaagaggtg gtacccgcac      60
   gccgaaatgc atgcaagtaa cctattcaaa gtaatatctc atacatgttt catgagggta     120
   acaacatgcg actgggtgag catatgttcc gctgatgtga tgtgcaagat aaacaagcaa     180
   ggcagaaact aacttcttct tcatgtaata aacacacccc gcgtttattt acctatctct     240
   aaacttcaac accttatatc ataactaata tttcttgaga taagcacact gcacccatac     300
   cttccttaaa aacgtagctt ccagtttttg gtggttccgg cttccttccc gattccgccc     360
   gctaaacgca tatttttgtt gcctggtggc atttgcaaaa tgcataacct atgcatttaa     420
   aagattatgt atgctcttct gacttttcgt gtgatgaggc tcgtggaaaa aatgaataat     480
   ttatgaattt gagaacaatt ttgtgttgtt acggtatttt actatggaat aatcaatcaa     540
   ttgaggattt tatgcaaata tcgtttgaat atttttccga ccctttgagt acttttcttc     600
   ataattgcat aatattgtcc gctgcccctt tttctgttag acggtgtctt gatctacttg     660
   ctatcgttca acaccacctt attttctaac tatttttttt ttagctcatt tgaatcagct     720
   tatggtgatg gcacattttt gcataaacct agctgtcctc gttgaacata ggaaaaaaaa     780
   atatataaac aaggctcttt cactctcctt gcaatcagat ttgggtttgt tccctttatt     840
   ttcatatttc ttgtcatatt cctttctcaa ttattatttt ctactcataa cctcacgcaa     900
   aataacacag tcaaatctag aattcggatc cggtagatac attgatgcta tcaatccaga     960
   gaactggaaa gattgtgtag ccttgaaaaa cggtgaaact tacgggtcca agattgtcta    1020
   cagattttcc tgatttgcca gcttactatc cttcttgaaa atatgcactc tatatctttt    1080
   agttcttaat tgcaacacat agatttgctg tataacgaat tttatgctat tttttaaatt    1140
   tggagttcag tgataaaagt gtcacagcga atttcctcac atgtagggac cgaattgttt    1200
   acaagttctc tgtaccacca tggagacatc aaaaattgaa aatctatgga aagatatgga    1260
   cggtagcaac aagaatatag cacgagccgc ggatttattt cgttacgcat gcgc          1314
<210> 43
   <211> 1014
   <212> DNA
   <213> Lactobacillus helveticus;
<400> 43
   atgacaaagg tttttgctta cgctattcga aaagacgaag aaccattctt gaatgaatgg     60
   aaggaagctc acaaggatat cgatgttgat tacactgata aacttttgac tcctgaaact    120
   gctaagctag ctaagggtgc tgacggtgtt gttgtttacc aacaattaga ctacactgca    180
   gatactcttc aagctttagc agacgctggc gtaactaaga tgtcattacg taacgttggt    240
   gttgacaaca ttgatatgga caaggctaag gaattaggtt tccaaattac caatgttcct    300
   gtttactcac caaacgctat tgctgaacat gctgctattc aggctgcacg tgtattacgt    360
   caagacaagc gcatggacga aaagatggct aaacgtgact tacgttgggc accaactatc    420
   ggccgtgaag ttcgtgacca agttgtcggt gttgttggta ctggtcacat tggtcaagta    480
   tttatgcgta ttatggaagg tttcggtgca aaggttattg cttacgatat cttcaagaac    540
   ccagaacttg aaaagaaggg ttactacgtt gactcacttg acgacttgta caagcaagct    600
   gatgtaattt cacttcacgt accagatgtt ccagctaacg ttcacatgat caacgacaag    660
   tcaatcgctg aaatgaaaga cggcgttgta attgtaaact gctcacgtgg tcgacttgtt    720
   gacactgacg ctgtaatccg tggtttggac tcaggcaaga tcttcggctt cgttatggat    780
   acttacgaag acgaagttgg tgtatttaac aaggattggg aaggtaaaga attcccagac    840
   aagcgtttgg cagacttaat tgatcgtcca aacgtattgg taactccaca caccgccttc    900
   tacactactc acgctgtacg taacatggtt gttaaggcat tcaacaacaa cttgaagtta    960
   atcaacggcg aaaagccaga ttctccagtt gctttgaaca agaacaagtt ttaa         1014

## Description

### Background of the Invention

Lactic acid is an organic molecule that can be produced either by chemical synthesis or by fermentation processes in microorganisms (biosynthesis). Advantages that a biosynthetic approach may have over a chemical synthetic approach for manufacturing an organic product include more efficient yield of product, faster production, isomeric purity, and lower cost.

D-(or R-)lactic acid is a building block in the manufacture of biologically active materials, including herbicides and pharmaceuticals. However, new processes that lower the cost of D-lactic acid could enable its penetration and widespread use in larger chemical markets including, for example, plastics. D-lactic acid can be converted to D-lactide, a cyclic condensate of D-lactic acid, which has many potential applications, including the manufacture of films, fibers, and other polymer applications. D-lactide can be polymerized to poly(D-lactide) (D-PLA), a polymer that has properties similar to poly(L-lactide) (L-PLA), which is used for various polymer applications. Highly crystalline PLA as currently produced by Cargill Dow predominately contains the L-stereoisomer. This high L-isomer resin is most commonly used in the PLA fibers market because of its ability to increase fiber tenacity, resist shrinkage, and increase its useable temperature above the L-PLA glass transition temperature, when high stresses are applied to it. A polymer similarly high in D-isomer would be expected to have similar properties. Further, a high D-isomer polymer can be blended with a high L-isomer polymer to form a stereo-complex that has a significantly higher melting temperature (up to approximately 230°C). This higher melting temperature allows PLA to be used in applications where better heat resistance is needed. An example of such an application is in certain apparel applications, where an increased melting temperature is needed to produce ironable fabrics.

D-lactic acid is also a useful industrial chemical intermediate that is used in herbicide and pharmaceutical applications. For example, D-lactic acid is an intermediate in the manufacture of phenoxypropionic and aryloxyphenoxypropionic herbicides.

Known biosynthetic processes for producing lactic have certain limitations. For example, natural lactic acid producing organisms such as *Lactobacilli* can produce large quantities of L-lactic acid under fermentation conditions. However, these organisms require a complex fermentation medium in order to produce efficiently. The complexity of the fermentation medium increases raw material costs and makes it more difficult and expensive to separate the lactic acid from the medium. Further, fermentation processes using these organisms are prone to infection from other, non-lactic acid producing species. There is no economical method to selectively eliminate the unwanted strains.

It is necessary to maintain the pH in the fermentation broth when these organisms are used, as low pH environments both within the bacteria itself and in the broth can inhibit proliferation of the bacteria or cause cell death. This is accomplished by adding neutralizing agents such as calcium carbonate to the fermentation broth to form calcium lactate. In order to recover lactic acid, it is necessary to treat the calcium lactate with a strong acid such as sulfuric acid, which reacts with calcium lactate to form lactic acid and gypsum (calcium sulfate). The inability of these organisms to function at low pH therefore leads to additional expenses for raw materials and for disposal of unwanted by-products (gypsum).

In Japanese Kokai No. 2002-136293A there is disclosed a genetically engineered yeast which is said to produce D-lactic acid. The yeast host is of a special type that "accumulates" pyruvate, i.e., produces significant quantities of pyruvate that are not further metabolized.

Dequin et al. (BIO/TECHNOLOGY, 1994, vol 12, no. 2, pages 173-177) teach the expression of L-lactate dehydrogenase from Lactobacillus casei in the yeast Saccharomyces cerevisiae from plasmid. Similarly teach Chelstowska et al. (YEAST, 1999, vol. 15, no. 13, pages 1377-1391) the expression of yeast D-lactate dehydrogenase DLD2 and DLD3 from plasmid in the yeast S. cerevisiae.

Thus, there remains a need in the art for biosynthetic processes for preparing D-lactic acid with an organism that: 1) permits D-lactic acid to be made at good yields and productivities; 2) preferably requires only a simplified fermentation medium, and 3) preferably allows for a low pH and/or a high temperature fermentation medium that can eliminate contamination from unwanted microorganism strains.

### Summary Of The Invention

In one aspect, this invention provides recombinant yeast cells of a species that does not naturally accumulate pyruvate, comprising at least one exogenous D-lactate dehydrogenase gene integrated into its genome, wherein the D-lactate dehydrogenase gene is operatively linked to functional promoter and terminator sequences.

The invention also provides recombinant nucleic acids encoding a D-lactate dehydrogenase gene operatively linked to functional promoter and terminator sequences. The recombinant nucleic acids of the invention permit expression of the D-lactate dehydrogenase gene in a recombinant yeast cell.

In another aspect, the invention provides a method for producing D-lactic acid comprising fermenting cells of the invention under conditions that allow for the biosynthesis of D-lactic acid.

The transformed yeast cells are capable of producing D-lactic acid at commercially-significant yields. The cells can tolerate the presence of D-lactic acid well and can continue to produce efficiently when the fermentation broth contains significant concentration of D-lactic acid. This effect is unexpected, as both L-lactic and D-lactic acid tend to be inhibitory to the growth and survival of certain microorganisms (*see* Lett. Appl. Microbiol. 2002;35(3):176-80; Susceptibility of *Escherichia coli* 0157 and non-0157 isolates to lactate. McWilliam Leitch EC, Stewart CS. Appl. Environ. Microbiol. 2002 Sep;68(9):4676-8.). In some cases eukaryotic and bacterial cells have been shown to respond differently to D-lactic acid than to L-lactic acid (see Uribarri J, Oh MS, Carroll HJ, Medicine (Baltimore) 1998 Mar;77(2):73-82, "D-lactic acidosis: a review of clinical presentation, biochemical features, and pathophysiologic mechanisms;" cf. Leitch *et al, supra*).

Specific preferred embodiments of the present invention will become evident from the following more detailed description of certain preferred embodiments and the claims.

### Brief Description Of The Drawings

FIG. 1 is a plasmid map of pNC2, comprising the *PGK* promoter and *GAL10* terminator, both from *S. cerevisiae.*
FIG. 2 is a plasmid map of pNC4, comprising the *PDC1* promoter and *GAL10* terminator, both from *S. cerevisiae.*
FIG. 3a is a map of a *Nof*I restriction fragment of a 1.235 kb sequence derived from PCR amplified *S. cerevisiae* chromosomal DNA, that comprises the *PGK* promoter and *GAL10* terminator (*S. cerevisiae*), and a multiple cloning site between the promoter and terminator.
FIG. 3b is a map of *Not*I restriction fragment of a 1.235 kb sequence derived from PCR amplified *S. cerevisiae* chromosomal DNA, that comprises the *PDC1* promoter and *GAL10* terminator (*S. cerevisiae*), and a multiple cloning site between the promoter and terminator.
FIG. 4 is a plasmid map of pVR24, comprising the *B. megaterium L-LDH* functionally linked to the *PGK* promoter and *GAL10* terminator from *S. cerevisiae.*
FIG. 5 is a plasmid map of pVR22, comprising the G418 resistance marker functionally linked to the *PGK* promoter and *GAL10* terminator from *S. cerevisiae.*
FIG. 6 is a plasmid map of pNC7, comprising the *LDH* gene from *B. megaterium,* functionally linked to the *PGK* promoter and *GAL10* terminator from *S. cereirisiae.*
FIG. 7A-B are: A) a plasmid map ofpSO21, comprising *PDC1* from *K. marxianus;* and B) a plasmid map of pSO27, comprising a 3.3kb fragment of *PDC1 (K. marxianus)* that contains a 5' upstream region of the *PDC1* locus; and FIG. 8 is a plasmid map of pSO28, comprising a deletion in the *PDC1* coding region contained in pSO21, and the G418 resistance gene functionally linked to the *PGK* promoter and the *GAL10* terminator from *S. cerevisiae.*
FIG. 9 is a plasmid map of pSO29, comprising a deletion in the *PDC1* coding region contained in pSO21, and the zeocin resistance gene functionally linked to the *TEF1* promoter and the *Tcyc1* terminator from *S. cerevisiae* (from pTEF1/Zeo; Invitrogen).
FIG. 10 is a plasmid map of pPS1, comprising the hygromycin resistance gene (*hph*) from *E*. *coli* functionally linked to the *PDC1* promoter and *GALL10* terminator from *S. cerevisiae.*
FIG. 11a is a plasmid map of pVR43, comprising the *D-LDH* gene from *L. helveticus.*
FIG. 11b is a plasmid map of pVR44, comprising the *D-LDH* gene from *L. helveticus* with the *Xba*I and *Bam*HI sites at the 5' and 3' end of the gene respectively.
FIG. 12 is a plasmid map of pVR47, comprising the *D-LDH* gene from *L. helveticus* functionally linked to the *PGK* promoter and *GAL10* terminator from *S. cerevisiae.*
FIG. 13 is a plasmid map of pVR48, comprising the *D-LDH* gene from *L. helveticus* functionally linked to the *PGK* promoter and *GAL10* terminator from *S. cerevisiae,* and the *hph* resistance gene functionally linked to the *PDC1* promoter and the *GAL10* terminator from *S. cerevisiae.*
FIG. 14 is a plasmid map of pCA50, comprising an expression cassette inserted between 3' and 5' flanking regions of the *PDC1* locus (*K. marxianus),* containing the *D-LDH* gene from *L. helveticus* functionally linked to the *PGK* promoter and *GAL10* terminator from *S. cerevisiae,* and the *hph* resistance gene functionally linked to the *PDC1* promoter and *GAL10* terminator from S. *cerevisiae.*
FIG. 15 is a plasmid map of pVR29, comprising the G418 resistance marker functionally linked to the *PGK* promoter and *GAL10* terminator from *S. cerevisiae.*
FIG. 16a is a plasmid map of pBH5a, comprising a 5' flank of the *PDC1* locus (*K. marxianus*) and separately, the G418 resistance gene functionally linked to the *PDC1* promoter and *GAL10* terminator from *S. cerevisiae.*
FIG. 16b is a plasmid map of pBH5b, comprising 5' and 3' flanking regions of the *PDC1* locus (*K. marxianus*) and separately, the G418 resistance gene functionally linked to the *PDC1* promoter and *GAL10* terminator from *S. cerevisiae.*
FIG. 16C is a plasmid map of pBH5c, comprising the *PDC1* locus from *K. marxianus.*
FIG. 17 is a plasmid map of pBH6, comprising the *D-LDH* gene from *L. helveticus* located between 5' and 3' flanking regions of the *PDC1* locus (*K*. *marxianus*), and separately the G418 resistance gene functionally linked to the *PDC1* promoter and the *GAL10* terminator from S. *cerevisiae.*

### Detailed Description Of The Invention

The yeast cells of the invention are provided from a species that, prior to recombination as described herein, does not accumulate pyruvate. By "does not accumulate" pyruvate, it is meant that the species does not produce at least 10g/L of pyruvic acid when cultured in accordance with the method set forth in Embodiment 5 of Japanese Kokai 2000-78996. Generally, a yeast cell will not accumulate pyruvate when it naturally contains an active metabolic pathway that metabolizes the pyruvate to various metabolites such as ethanol or acetate or biomass. Such yeast cells metabolize the pyruvate rapidly enough that little pyruvate exists within the cell at any one time and little pyruvate is secreted by the cell. Preferred yeast cells naturally have one or more active pyruvate decarboxylase (PDC) genes that produce pyruvate decarboxylase protein, which converts pyruvate to ethanol. Even more preferred yeast cells are those that exhibit the Crabtree negative phenotype, in which the cell's aerobic metabolic pathway (respiration and TCA cycle) is not inhibited by the present of a high concentration of glucose. Examples of suitable yeast cells include those from the genera *Candida, Saccharomyces, Kluyveromyces, Pichia, Hansenula.* Cells from the genera *Candida* and *Kluyveromyces* are particularly preferred. Especially preferred cells are *C. sonorensis, K. lactis, K. theromotolerans* and *K. marxianus.* Most preferred cells are *C. sonorensis* and *K. marxianus.*

The recombinant yeast cells of the invention contain at least one exogenous D-LDH gene integrated into its genome. *Lactobacillus helveticus, Lactobacillus johnsonii, Lactobacillus bulgaricus, Lactobacillus delbrueckii, lactobacillus plantarum and Lactobacillus pentosus* are strains that have suitable D-lactate dehydrogenases that can be obtained by, *inter alia*, recombinant genetic techniques for use herein. A preferred D-lactate dehydrogenase gene is *L. helveticus* D-lactate dehydrogenase. The cell may contain multiple exogenous LDH genes, *i.e.*, at least two such genes, preferably about 2-10 of such genes, more preferably 2-5 of such genes. The inserted LDH genes may be all the same gene, or may be comprised of two or more different types of LDH gene.

A gene, promoter or terminator is considered to be "exogenous" for purposes of this invention if it (1) is not found within the genome of the unmodified cell, and (2) is not homologous to genetic material present in the genome of the unmodified cell. As used herein, a gene, terminator or promoter is "native" to the yeast species if it is found (apart from individual-to-individual mutations which do not effect its function) within the genome of the unmodified cells of that species of yeast.

The exogenous D-LDH gene is operatively linked to functional promoter and terminator sequences. By "operatively linked" it is meant, within the context of this invention, that the promoter or terminator, as the case may be, functions after integration in the yeast genome to control the start and end, respectively, of transcription of the D-LDH gene.

As used herein, the term "promoter" refers to an untranscribed sequence located upstream (*i.e.*, 5') to the translation start codon of a structural gene (generally within about 1 to 1000 bp, preferably 1-500 bp, especially 1-100 bp) and which controls the start of transcription of a structural gene. The promoter may be native to the host cell or exogenous. The promoters can be those that control the expression of genes that are involved in central carbon metabolism, *e.g.*, glycolytic promoters or TCA cycle gene promoters. Suitable promoters include the non-limiting examples of promoters from yeast genes phosphoglycerate kinase (*PGK*), glyceraldehyde-3-phosphate dehydrogenase (*TDH*), pyruvate decarboxylase *(PDC1),* triose phosphate isomerase (*TP1*), Transcription enhancer factor-1 (*TEF-1*), purine-cytosine permease (*PCPL3*), and alcohol dehydrogenase *(ADH).* Preferred promoters of the invention include the *TEF*-*1 (S. cerevisiae), PGK (S. cerevisiae),* and *PDCl (S. cerevisiae, K. marxianus)* promoters.

In embodiments where it is desired to integrate the D-LDH gene(s) at a targeted locus of the yeast cell's genome, the promoter sequence is homologous to the promoter sequence of the gene where insertion is targeted. The targeted gene is preferably a pyruvate decarboxylase (PDC) gene. Additional advantageous target genes include alcohol dehydrogenase (ADH), orotidine-5'-phosphate decarboxylase (*ura3*), and 3-isopropylmalate dehydrogenase (*leu2*).

Similarly, the term "terminator" refers to an untranscribed sequence located downstream (*i.e.*, 3') to the translation finish codon of a structural gene (generally within about 1 to 1000 bp, more typically 1-500 base pairs and especially 1-100 base pairs) and which controls the end of transcription of the structural gene. The terminator can be exogenous or native to the yeast species. Suitable exogenous terminators include the GAL10 and CYC-1 terminators from *S. cerevisae* or other yeast species.

As with the promoters, in certain embodiments, the terminator sequence is homologous to a terminator sequence of the targeted gene.

A gene, promoter, terminator or other genomic material is considered to be "homologous" to other genetic material if it is identical, i.e. has at least 90%, 91%, 92%, 93%, 94%, 95%, 96%, 97%, 98%, or about 99% identity in nucleotide sequence to the other genetic material or if not identical, is sufficiently similar to it that it retains its function. Therefore, genetic material is considered to be "homologous" even if contains differences due to, e.g., point mutations, deletions or additions of base pairs, provided that those mutations, deletions or additions that do not affect the function of the genetic material. In the case of flanking sequences, homology is established if the sequence is similar enough to a flanking sequence of the native gene that the flanking sequence can each engage in a single crossover event with the flanking sequence of the native gene.

The term "identity," as known in the art, refers to a relationship between the sequences of two or more polypeptide molecules or two or more nucleic acid molecules, as determined by comparing the sequences thereof. In the art, "identity" also means the degree of sequence relatedness between nucleic acid molecules or polypeptides, as the case may be, as determined by the match between strings of two or more nucleotide or two or more amino acid sequences. "Identity" measures the percent of identical matches between the smaller of two or more sequences with gap alignments (if any) addressed by a particular mathematical model or computer program (*i.e.*, "algorithms").

The term "similarity" is used in the art with regard to a related concept, but in contrast to "identity," "similarity" refers to a measure of relatedness, which includes both identical matches and conservative substitution matches. If two polypeptide sequences have, for example, 10/20 identical amino acids, and the remainder are all non-conservative substitutions, then the percent identity and similarity would both be 50%. If in the same example, there are five more positions where there are conservative substitutions, then the percent identity remains 50%, but the percent similarity would be 75% (15/20). Therefore, in cases where there are conservative substitutions, the percent similarity between two polypeptides will be higher than the percent identity between those two polypeptides.

Identity and similarity of related nucleic acids and polypeptides can be readily calculated by known methods. Such methods include, but are not limited to, those described in COMPUTATIONAL MOLECULAR BIOLOGY, (Lesk, A.M., ed.), 1988, Oxford University Press, New York; BIOCOMPUTING: INFORMATICS AND GENOME PROJECTS, (Smith, D.W., ed.), 1993, Academic Press, New York; COMPUTER ANALYSIS OF SEQUENCE DATA, Part 1, (Griffin, A.M., and Griffin, H.G., eds.), 1994, Humana Press, New Jersey; von Heinje, G., SEQUENCE ANALYSIS IN MOLECULAR BIOLOGY, 1987, Academic Press; SEQUENCE ANALYSIS PRIMER, (Gribskov, M. and Devereux, J., eds.), 1991, M. Stockton Press, New York; Carillo et al., 1988, SIAM J. Applied Math., 48:1073; and Durbin *et al.*, 1998, BIOLOGICAL SEQUENCE ANALYSIS, Cambridge University Press.

Preferred methods to determine identity are designed to give the largest match between the sequences tested. Methods to determine identity are described in publicly available computer programs. Preferred computer program methods to determine identity between two sequences include, but are not limited to, the GCG program package, including GAP (Devereux et al., 1984, Nucl. Acid. Res., 12:387; Genetics Computer Group, University of Wisconsin, Madison, WI), BLASTP, BLASTN, and FASTA (Altschul et al., 1990, J. Mol. Biol., 215:403-410). The BLASTX program is publicly available from the National Center for Biotechnology Information (NCBI) and other sources (BLAST Manual, Altschul et al. NCB/NLM/NIH Bethesda, MD 20894; Altschul *et al.*, 1990, supra). The well-known Smith Waterman algorithm may also be used to determine identity.

Certain alignment schemes for aligning two amino acid or polynucleotide sequences may result in matching of only a short region of the two sequences, and this small aligned region may have very high sequence identity even though there is no significant relationship between the two full-length sequences. Accordingly, in certain embodiments, the selected alignment method (GAP program) will result in an alignment that spans at least 50 contiguous amino acids of the target polypeptide. In some embodiments, the alignment can comprise at least 60, 70, 80, 90, 100, 110, or 120 amino acids of the target polypeptide. If polynucleotides are aligned using GAP, the alignment can span at least about 100, 150, or 200 nucleotides, which can be contiguous.

For example, using the computer algorithm GAP (Genetics Computer Group, University of Wisconsin, Madison, WI), two polypeptides for which the percent sequence identity is to be determined are aligned for optimal matching of their respective amino acids (the "matched span", as determined by the algorithm). In certain embodiments, a gap opening penalty (which is calculated as three-times the average diagonal; where the "average diagonal" is the average of the diagonal of the comparison matrix being used; the "diagonal" is the score or number assigned to each perfect amino acid match by the particular comparison matrix) and a gap extension penalty (which is usually one-tenth of the gap opening penalty), as well as a comparison matrix such as PAM250 or BLOSUM 62 are used in conjunction with the algorithm. In certain embodiments, a standard comparison matrix (see Dayhoff et al., 1978, Atlas of Protein Sequence and Structure, 5:345-352 for the PAM 250 comparison matrix; Henikoff et al., 1992, Proc. Natl. Acad. Sci USA, 89:10915-10919 for the BLOSUM 62 comparison matrix) is also used by the algorithm.

In certain embodiments, the parameters for a polypeptide sequence comparison include the following:
Algorithm: Needleman et al., 1970, J. Mol. Biol., 48:443-453;
Comparison matrix: BLOSUM 62 from Henikoff *et al*., 1992, *supra;*
Gap Penalty: 12
Gap Length Penalty: 4
Threshold of Similarity: 0
The GAP program may be useful with the above parameters. For nucleotide sequences, parameters can include a gap penalty of 50 and a gap length penalty of 3, which is a penalty of 3 for each symbol in each gap. In certain embodiments, the aforementioned parameters are the default parameters for polypeptide comparisons (along with no penalty for end gaps) using the GAP algorithm.

The yeast cell may have various other modifications to its natural genome. For example, the yeast cell may contain various selection marker genes, as described more below, together with associated promoter and/or terminator sequences. The yeast cell may further have a deleted PDC gene or a disruption in a PDC gene. A method of deleting the PDC together with the insertion of the D-LDH gene at the locus of the PDC gene is described more fully below. Other methods of deleting or disrupting PDC activity are described in Porro, "Development of metabolically engineered Saccharomyces cerevisiae cells for the production of lactic acid", Biotechnol. Prog. 1995 May-Jun; 11(3): 294-8; Porro et al., "Replacement of a metabolic pathway for large-scale production of lactic acid from engineered yeasts", App. Environ. Microbiol. 1999 Sep:65(9):4211-5; Bianchi et al., "Efficient homolactic fermentation by Kluyveromyces lactis strains defective in pyruvate utilization and transformed with the heterologous LDH gene", App. Environ. Microbiol. 2001 Dec; 67(12)5621-5; and WO 99/14335.

The recombinant yeast cells of the invention can be prepared by insertion of a nucleic acid fragment containing the D-LDH gene operatively linked to a promoter sequence. The fragment typically forms a part of a recombinant nucleic acid which can and preferably does contain other elements as well, including (a) a terminator sequence; (b) one or more selection marker gene(s) (including an associated promoter and terminator); (c) one or more homologous flanking sequences for inserting the fragment at a particular locus in the genome of the host cell; (d) one or more restriction sites which enable it to be cut to form a linear fragment containing the LDH gene, its promoters and flanking sequences, marker genes and associated promoters and terminators, etc., for insertion into the genome of the yeast cell; and/or (e) a backbone portion. The term "recombinant nucleic acid" is used herein to refer to any molecule (*e.g.*, nucleic acid, plasmid or virus) used to transfer protein-coding information to the host cell. Methods of transforming cells are well known in the art, and can include such non-limiting examples as electroporation, calcium chloride-, or lithium acetate-based methods. The DNA used in the transformations can either be cut with particular restriction enzymes or uncut.

As used herein, the term "selection marker genes" refer to genetic material that encodes a protein necessary for the survival and/or growth of a host cell grown in a selective culture medium. Typical selection marker genes encode proteins that (a) confer resistance to antibiotics or other toxins, *e.g.,* zeocin (sh ble gene from *Streptoalloteichus hindustanus),* G418 (kanamycin-resistance gene of Tn903), hygromycin (aminoglycoside antibiotic resistance gene from *E. coli*), ampicillin, tetracycline, or kanamycin for host cells; (b) complement auxotrophic deficiencies of the cell and/or supply critical nutrients not available from simple media, such as amino acid leucine deficiency (*K. marxianus* Leu2 gene); or a *K. marxianus* ura3 gene that gives uracil to orotidine-5'-phosphate decarboxylase negative cells. Preferred selectable markers include the non-limiting examples of zeocin resistance gene, G418 resistance gene, and the hygromycin resistance gene.

Backbone portions are conveniently obtained from commercially available yeast vectors.

Suitable methods of transforming yeast cells to insert an exogenous LDH gene are described in WO 00/71738A1 and WO 02/42471A1. The methods described there are generally applicable to make the recombinant yeast cells of this invention, with the substitution of a D-LDH gene for the L-LDH genes described there.

The terms "transforming" and "transformation" as used herein refers to a change in a cell's genetic characteristics, and a cell has been transformed when it has been modified to contain a new nucleic acid. Thus, a cell is transformed where it is genetically modified from its native state. For example, following transfection, transforming DNA preferably recombines with cellular genomic DNA by physically integrating into a chromosome of the cell. Alternatively, at least transiently (i.e., within 48-96 hrs of cellular transformation), the nucleic acid can be maintained transiently as an episomal element without being replicated, or may replicate independently as a plasmid. A cell is considered to have been stably transformed when the DNA is integrated into the chromosome and is replicated with the division of the cell.

The term "transfection" is used to refer to the uptake of foreign or exogenous DNA by α cell, and a cell has been "transfected" when the exogenous DNA has been introduced inside the cell membrane. A number of transfection techniques are well known in the art. *See, e.g.*, Graham et al., 1973. Virology 52:456; Sambrook et al., 2001, MOLECULAR CLONING, A LABORATORY MANUAL, Cold Spring Harbor Laboratories; Davis et al., 1986, BASIC METHODS IN MOLECULAR BIOLOGY, Elsevier; and Chu et al., 1981, Gene 13:197. Such techniques can be used to introduce one or more exogenous DNA species into suitable host cells.

Multiple D-LDH genes can be integrated by multiple transformations. However, it is possible to construct a recombinant nucleic acid containing multiple D-LDH genes, thus enabling multiple LDH genes to be inserted in a single step.

A transformation method of particular interest targets the insertion of the D-LDH gene at the locus of a naturally-occurring target gene. A target gene is any gene that is desired to be replaced with the LDH gene. A preferred target gene is a pyruvate decarboxylase gene, as replacing this gene disrupts a competing pathway that produces ethanol. In addition, the pyruvate gene tends to be a active in yeast species, so insertion of the LDH gene into the genome under control of the PDC promoters and terminators tends to produce a mutant that expresses LDH well. Additional preferred target genes are ADH, Leu2 and Ura3.

Preferably, the yeast cells of the invention are transformed with recombinant nucleic acids of the invention and selected by growth in a selective medium in which the selectable marker in the recombinant nucleic acid permits preferential growth of transformants. The transformed cells are preferably grown after selection under non-selective conditions. Under these conditions, recombinant yeast cells are obtained having the exogenous D-LDH gene comprising the recombinant nucleic acid integrated into the genetic locus of the target gene in the yeast chromosome. As obtained according to the methods of this invention, these cells have deleted the target gene, and the integrated, exogenous D-LDH gene is inserted into the target gene locus so that it is operably linked and under the transcriptional control of the expression control sequences (such as the promoter and terminator sequences) of the target gene. When the target gene is a PDC gene, cells in which the PDC deletion occurs do not grow well under anaerobic conditions. Thus, colonies of the identified and selected cells can be selected by exposing them to anaerobic conditions. Colonies that do not grow are identified as those in which the PDC deletion has occurred. Similarly, targeted integration into any other target gene can be identified by the phenotype associated with deletion of each of the target genes.

The resulting yeast cell is missing the target gene, and contains an exogenous D-LDH gene integrated into the yeast cell genome at the locus of the target gene. The LDH gene is under the transcriptional control of a promoter sequence and a terminator sequence that is homologous to promoter and terminator sequences of the target gene.

The D-LDH promoter and terminator sequences may be those which were present in flanking sequences contained in the recombinant nucleic acid that was used to transform the cell, or may be those which were originally present in the cell's genome at the site of integration. It is also possible that the target gene terminator is retained with deletion of the terminator sequence that was present in the integration vector.

The flanking sequence may be immediately adjacent to the D-LDH gene, or separated from the gene by an intermediate sequence of base pairs, such as from 1-1000, preferably 1-100 base pairs. The flanking sequences used in the recombinant nucleic acids of this invention are preferanbly homologous to corresponding flanking sequences of the targeted gene. Typical flanking sequences lengths are from about 50 to about 4000 base pairs, preferably about 100 to about 2000 base pairs, especially up to about 1200 base pairs. The flanking sequences preferably contain a promoter (terminator in the case of a downstream flanking sequence) sequence each homologous to those the target gene. In preferred embodiments, the flanking sequences are homologous to and comprise promoter and terminator sequences, respectively, for native yeast Most preferably, integration of the recombinant nucleic acid into the target gene locus in the chromosomal DNA of the yeast genome results in the exogenous D-LDH gene encoded thereby to fall under the transcription control of the native gene expression regulatory sequences comprising said flanking sequences.

Suitable flanking sequences can be obtained by identifying the intended site of integration in the yeast cell genome, cloning the sequences that flank that site (using any convenient method) and attaching those sequences into the desired position in the recombinant nucleic acid.

The recombinant nucleic acid further preferably includes one or more selection marker genes, which are more preferably under the transcriptional control of their own promoter and terminator sequences. In this embodiment, promoter and terminator sequences for marker genes are preferably not promoter and terminator sequences for the target gene. The selection marker gene(s) and their respective promoters and terminators preferably do not interrupt the sequence of upstream flanking sequence-LDH gene-downstream flanking sequence of the recombinant nucleic acid. The selection marker gene(s) and respective promoters and terminators are preferentially positioned on the recombinant nucleic acid upstream (5') to the upstream flanking sequence.

A target gene is any gene that is desired to be replaced with the LDH gene. A preferred target gene is a pyruvate decarboxylase gene, as replacing this gene disrupts a competing pathway that produces ethanol. In addition, the pyruvate gene tends to be a active in yeast species, so insertion of the LDH gene into the genome under control of the PDC promoters and terminators tends to produce a mutant that expresses LDH well. Additional preferred target genes include alcohol dehydrogenase (ADH), orotidine-5'-phosphate decarboxylase (ura3), and 3-isopropylmalate dehydrogenase (leu2).

The resulting yeast cell is missing the target gene, and contains an exogenous D-LDH gene integrated into the yeast cell genome at the locus of the target gene. The D-LDH gene is under the transcriptional control of a promoter sequence and a terminator sequence that are each homologous to promoter and terminator sequences, respectively, of the target gene.

In this embodiment, the LDH promoter and terminator sequences may be those which were present in the recombinant nucleic acid that was used to transform the cell, or may be those which were originally present in the cell's genome at the site of integration. After the first crossover event , the inserted D-LDH gene is operatively linked to promoters and terminator sequences that were present in the integration vector. After the second crossover event, either or both of those promoter and terminator sequences may be replaced with the native PDC promoter and/or terminator. For example, the native PDC promoter may be retained with deletion of the promoter sequence is that supplied with the integration vector. It is also possible that the native PDC terminator is retained with deletion of the terminator sequence that was present in the integration vector.

The transformed yeast cell of the invention is useful for producing D-lactic acid from sugars in a fermentation process. The cell preferably contains no functioning L-LDH gene, or if it does contain a functioning L-LDH gene, its activity is such that at least 90%, preferably at least 95%, more preferably at least 99.0%, even more preferably at least 99.5% of the lactic acid produced by the cell is the D-isomer.

The fermentation can be conducted using any convenient fermentation method. Typically the cell is provided with a carbohydrate that it is capable of metabolizing to pyruvate, and exposed to conditions under which fermentation occurs. The fermentation medium also contains nutrients (such as sources of nitrogen, phosphorus, sulfur, trace minerals, etc.) that promote the viability of the cells.

The particular carbohydrates that can be used depend on the particular host cell, and whether the host cell has been engineered to metabolize any particular carbohydrate to pyruvate. Hexose sugars such as glucose and fructose, oligomers of glucose such as maltose, isomaltose, maltotriose, starch, and sucrose, maltodextrins and xylose (a pentose sugar) are preferred.. Less preferred carbohydrates include galactose, mannose and arabinose.

The temperature during fermentation can be from about room temperature, more preferably from about 30°C, more preferably from about 35°C, to about 55°C, more preferably to about 50°C, even more preferably to about 45°C. The maximum temperature will depend somewhat on the particular host cell. When the host cell is *K. marxianus*, for example, the recombinant cell can tolerate relatively high temperatures (such as above 40°C and up to 50°C, especially up to 45°C). Another preferred host species, *C. sonorensis,* can tolerate temperatures up to about 40°C. This high temperature tolerance provides for the possibility of conducting the fermentation at such higher temperatures (thereby reducing cooling costs) without a significant loss of productivity. Another advantage provided by the good high temperature tolerance is that if the fermentation becomes contaminated with an undesired microorganism, in many cases the undesired microorganism can be selectively killed off by heating the fermentation medium to 40°C or more, especially 45°C or more, without significantly harming the desired cells of the invention.

During fermentation, the concentration of cells in the fermentation medium is typically in the range of about 1-150, preferably about 3-10, even more preferably about 3-6 g dry cells/liter of fermentation medium.

During the production phase of the fermentation, in some instances it may be preferred to operate microaerobically rather than strictly anaerobically. Optimum aeration conditions can be established for each microorganism by measuring specific oxygen uptake rates (OUR) and correlating those rates with yield, substrate consumption rates and the rate at which the desired fermentation product is produced. In many cases, yield and rates are optimized within a particular range of OUR. For yeast having a PDC disruption, optimum OUR values tend to be within the range of about 0.8 to about 3.5 mmol O₂/dry weight cells/hr. OUR refers to the rate at which oxygen is consumed by the cells during fermentation, and is expressed in units (mmoles or grams) of oxygen per dry weight of cells per unit time, such as mmol O₂/dry weight cells/hour. Oxygen consumption is conveniently determined by measuring oxygen introduced into the fermentation and oxygen removed from the fermentation. OUR measurements can be used as a basis to control aeration conditions (notably rate of gas introduction, agitation, proportion of oxygen in the aerating gas, etc.) during the production phase of a fermentation in order to maintain OUR within the range that is optimum for the particular organism. The concentration of dissolved oxygen in the broth is simultaneously maintained at less than 1% of saturation, particularly less than 10 micromol O₂/L. In a particularly preferred process, a growth phase of the ferementation is conducted such that the concentration of dissolved oxygen in the broth is reduced to less than 1% of saturation, particularly less than 10 micromol O₂/L, for a period of time, such as about 15-90 minutes, prior to the start of the production phase (i.e., switching from aerobic conditions in the growth phase to microaerobic conditions in the production phase.

As D-lactic acid is produced, the pH of the fermentation medium tends to drop unless a base is added to neutralize all or part of the acid as it forms. In one embodiment of the fermentation process, a neutralizing agent such as calcium carbonate, calcium hydroxide, sodium carbonate, sodium hydroxide, ammonia, ammonium hydroxide, and the like is added to the fermentation broth to maintain the pH within a desired range, typically from about 5.0 to about 8.0, especially from about 5.5 to about 7.5. When such a base is added, the corresponding lactate salt is formed. Recovery of the lactic acid therefore involves regenerating the free lactic acid. This is typically done by separating the cells and acidulating the fermentation broth with a strong acid such as sulfuric acid. A salt byproduct is formed (gypsum in the case where a calcium salt is the neutralizing agent and sulfuric acid is the acidulating agent), which is separated from the lactic acid. The lactic acid is then recovered through techniques such as liquid-liquid extraction, distillation, absorption, etc., such as are described in T.B. Vickroy, Vol. 3, Chapter 38 of Comprehensive Biotechnology, (ed. M. Moo-Young), Pergamon, Oxford, 1985; R. Datta, et al., FEMS Microbiol. Rev., 1995; 16:221-231; U.S. Patent Nos. 4,275,234, 4,771,001, 5,132,456, 5,420,304, 5,510,526, 5,641,406, and 5,831,122, and International Patent Application No: WO 93/00440.

Alternatively, the pH of the fermentation may be permitted to drop as lactic acid is produced by the cells. Thus, the pH of the fermentation broth may come within the range of about 1.5 to about 5.0, preferably from about 1.5 to about 4.2, more preferably from about 1.5 to about 3.86 (the pKa of lactic acid), especially from about 2.0 to below 3.86 due to the production of lactic acid. Conducting the fermentation in this manner can provide several benefits, if acceptable productivity and yields are achieved. Costs for neutralizing agents are reduced or eliminated. If the fermentation pH (at the end of the fermentation) is below the pKa of lactic acid, the lactic acid will exist mainly in the acid form. This allows the acidulation step to be eliminated, saving additional process steps, acidulation costs, and disposal costs for salt by-products. Thus, an especially preferred process includes continuing the fermentation until the pH of the fermentation broth falls below 3.86. Lactic acid can be separated from the resulting fermentation broth using methods such as those disclosed in WO 99/19290.

The ability of the cell to withstand a low pH environment provides another mechanism by which contamination from unwanted microorganisms can be eliminated. The culture containing the cell of the invention may be subjected to reduced pH conditions, such as a pH of from about 1.5-4.2, preferably from about 2.0 to 3.86, for a time sufficient to kill off contaminating microorganisms that are not acid-tolerant.

To be commercially useful, the recombinant yeast of the invention should exhibit several characteristics. The yeast should convert a significant proportion of the carbohydrate to lactic acid (i.e., produce a high yield of product). It should exhibit a high specific productivity, i.e., product a high amount of lactic acid per weight of cell per unit time. It preferably is tolerant to a fermentation pH below about 5.0, preferably from about 1.5 to 4.2, especially from 2.0 to 3.86, while providing good yields and productivities under those conditions. The cell is preferably also tolerant to high concentrations of D-lactic acid and/or D-lactic acid salts, at pH values of 5.0-8.0 and preferably at pH values from 1.5 to 5.0, preferably from 1.5 to 4.2 and especially from 2.0 to 3.86. This last property allows the fermentation process to use high concentrations of the starting carbohydrate.

In general, it is desirable that the fermentation process employing the recombinant cell of the invention provides some or all of the following features:
A. A yield of at least 30, preferably at least 40, more preferably at least 60, even more preferably at least 75 grams of lactic acid per gram of carbohydrate. The theoretical desired yield is 100%, but practical limits on yields are about 98% (g/g)B. A specific productivity of at least 0.1, preferably at least 0.3, more preferably at least about 0.4, especially at least about 0.5 grams of D-lactic acid/gram of cells/hour. Specific productivities are desirably as high as possible.
C. A titer (maximum concentration of D-lactic acid) of at least 15 grams/liter of fermentation medium, preferably at least 20 g/L, more preferably at least 40 g/L, even more preferably at least 80 g/L, up to 150 g/L, preferably up to about 120 g/L. The temperature of the fermentation medium affects the high end of readily achievable titers somewhat, as highly concentrated lactic acid solutions (*i.e.*, above about 150 g/liter) tend to become very viscous or gel at temperatures below about 35°C. Using a higher fermentation temperature, such as from about 35-50°C, permits higher titers without gelling or undue viscosity build-up.

Recombinant cells of the invention have been found to provide yields as high as 92-98% %, volumetric productivities of 1.5-2.2 g/L/hr and titers of 81-90 g/L when used in a neutral (pH 5.0-8.0) fermentation on glucose. At low pH fermentations, in which the pH is allowed to drop to about 3.0, cells of the invention have been found to provide yields of 80% or more and titers of 1.2-3.3 g/L. In all cases these results have been obtained without optimization of fermentation conditions.

In addition, the fermentation process of the invention preferably achieves a high volume productivity. Volume productivity is expressed as amount of product produced per unit volume of fermentation medium per unit time, typically gram of product/liter medium/hr of time. Volume productivities of at least 1.5 g/L/hr, preferably at least 2.0 g/L/hr, more preferably at least 2.5 g/L/hr are desirable. At preferred cell densities of up to 3-6 g cells/liter of fermentation medium, maximum productivities tend to up to about 5.0 g/L/hr, and more typically up to about 4.0 g/L/hr. It is highly preferred to conduct the fermentation so that these volume productivities are achieved when the medium pH, temperature, or both are within the ranges described in the preceding paragraph.

Lactic acid produced according to the invention is useful to produce lactide, a cyclic anhydride of two lactic acid molecules. Depending on the stereoisomer of the lactic acid, the lactide may be D-lactide (made from two D-lactic acid molecules), L-lactide (made from two L-lactic acid molecules) or D-L-lactide (made from one of each L-lactic acid and D-lactic acid molecules). A convenient method of producing lactide from lactic acid is via a polymerization/depolymerization method as described in USP 5,142,023 to Gruber et al.

Lactide, in turn, is particularly useful as a monomer for the production of polylactide polymers (PLA) and copolymers. Processes for preparing these are also described in USP 5,142,023 to Gruber et al. Preferred PLA products are melt-stable polymers as described in USP 5,338,822 to Gruber et al. The PLA may be semi-crystalline or amorphous.

The following examples serve to illustrate certain embodiments of the invention and do not limit it in scope or spirit.

### Examples

### Example 1A: Construction of expression plasmids pNC2, based on S. cerevisiae PGK promoter, and pNC4, based on S. cerevisiae PDC1 promoter.

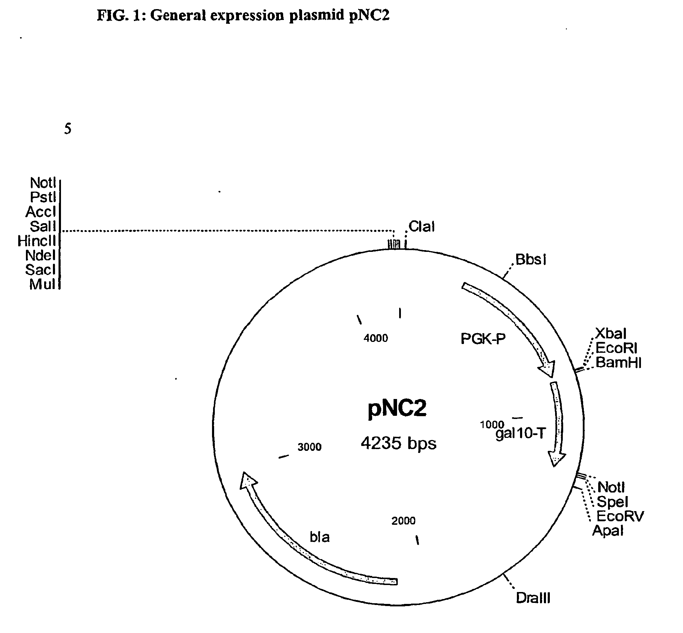

Expression plasmid pNC2 (FIG 1,) was generated by combining the S. *cerevisiae PGK1* promoter and the *S. cerevisiae GAL10* terminator on the pGEMSZ(+) (Promega, Wisconsin) backbone vector. The S. *cerevisiae PGK*1 promoter and the *GAL10* terminator were separated by a poly-linker region with the restriction sites *Xba*1*, EcoR*1 and *BamH*1 for inserting particular genes to be expressed between the yeast promoter and terminator.

A *S. cerevisiae PGK1* promoter having the following sequences was used: It was obtained as a restriction fragment from plasmid pBFY004. Alternatively, it can be obtained by PCR amplification using *S. cerevisiae* chromosomal DNA as template and primers designed based on the sequence:
The S. *cerevisiae GAL10* terminator used has the following sequence This sequence was obtained as a restriction fragment from plasmid pBFY004. Alternatively, it can be obtained by PCR amplification using *S. cerevisiae* chromosomal DNA as template and primers designed based on the sequence:

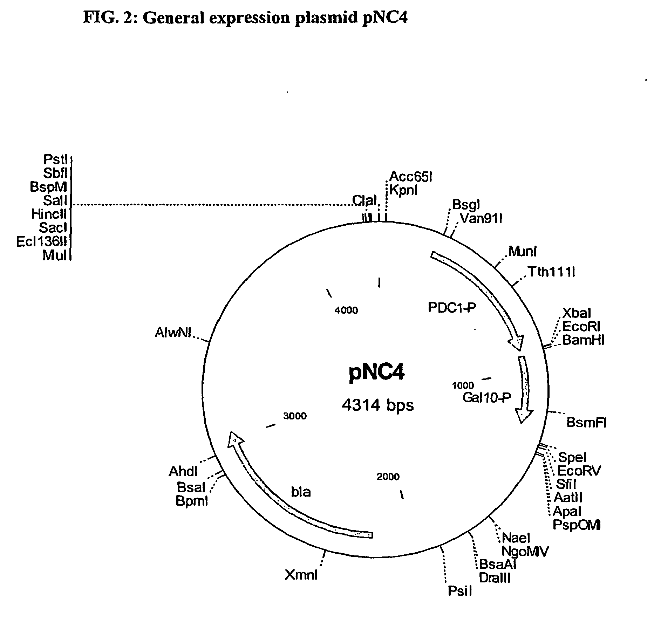

The plasmid pNC4 containing expression cassette based on *S. ceretrisiae PDC1* promoter and *GAL10* terminator was constructed and used as a general expression vector. Many of the marker genes have been expressed under this promoter. The pNC4 plasmid is shown in FIG. 2.

The plasmid backbone of pNC4 is pGEM5Z(+) (Promega Corporation; Madison, WI). The *S. cerevisiae PDC1* promoter was PCR amplified using the primers PSPDCS1 (5'-CCA TCG ATA ACA AGC TCA TGC AAA GAG-3'; SEQ ID No:3) and PSPDCAS2 (5'-GCT CTA GAT TTG ACT GTG TTA TTT TGCG-3'; SEQ ID No:4) and using chromosomal DNA from *S. cerevisiae* strain GY5098 as the template. Thermocycling was performed by 30 cycles of 1 min. at 94°C, 1 min. at 56°C, 1 min. at 72°C, followed by a final incubation of 7 min. at 72°C using PfuTurbo DNA polymerase (Stratagene).

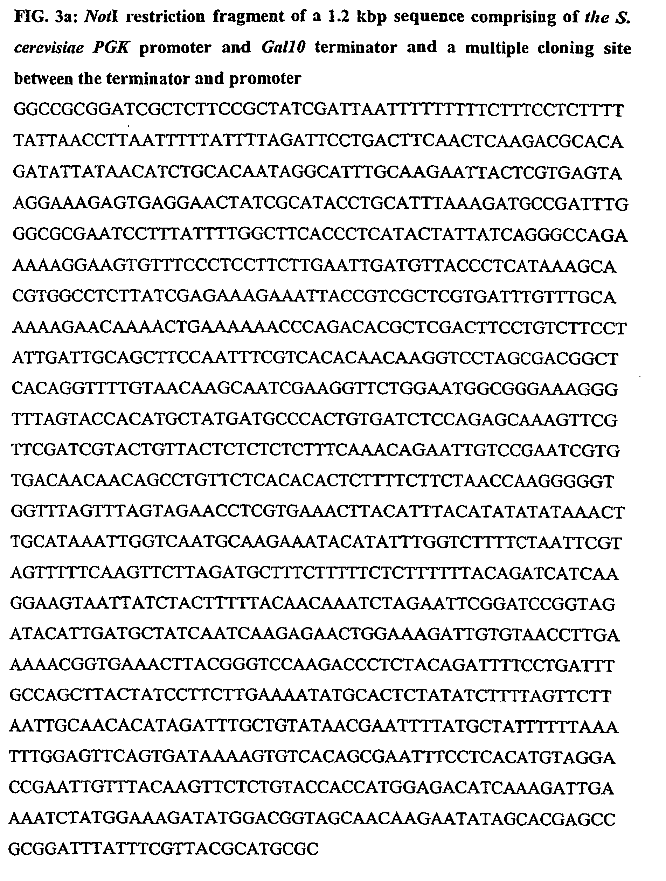

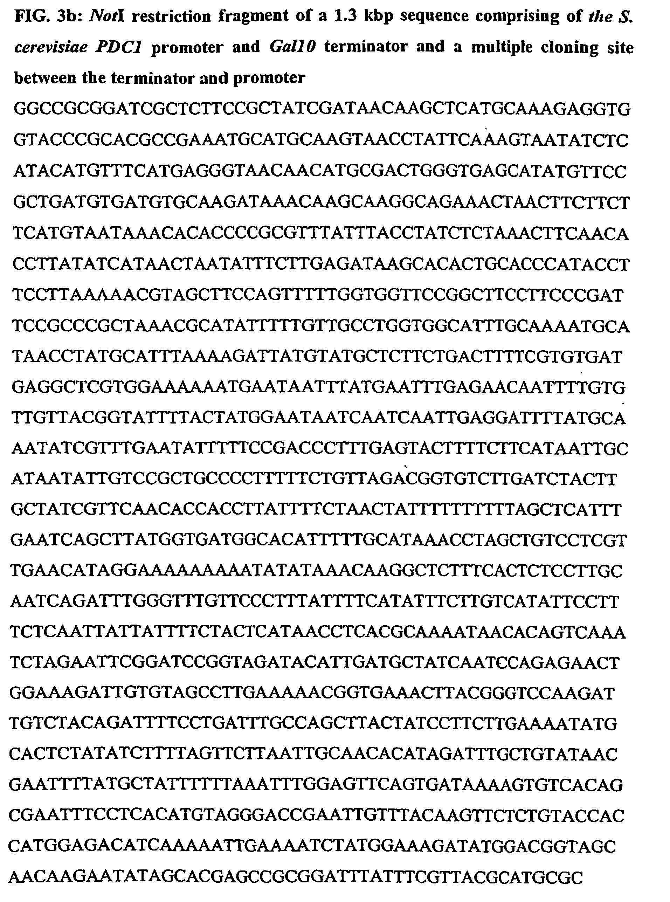

The *S. cerevisiae GAL10* terminator was obtained as described above. Fig. 3A SEQ ID NO 41 and 3B SEQ ID NO 42 depict the fragment comprising the *PGK1* promoter and *GAL10* terminator with multi-cloning sites, and the *PDC1* promoter and *GAL10* terminator with multi-cloning sites.

### Example 1B: Construction of pVR24 containing B. megaterium L-LDH under the control of the S. cerevisiae PGK1 promoter.

*B. megaterium* DNA encoding the *L-LDH* gene was isolated as follows. *B. megaterium* was obtained from the American Type Culture Collection (ATCC Accession #6458) and grown under standard conditions. Genomic DNA was purified from these cells using an Invitrogen "Easy-DNA" kit according to the manufacturer's protocol. Primers were designed on the basis of the available sequence in Genbank for the *L-LDH* from *B. megaterium* (Genbank accession #M22305). PCR amplification reactions were performed using Perkin Elmer buffer II (1.5 mM MgCl₂) and AmpliTaq Gold polymerase. Each reaction contained *B. megaterium* genomic DNA at a concentration of 6 ng/µL, each of 4 dNTPs at a concentration of 0.2 mM, and each of two amplification primers BM1270 and BM179 at a concentration of 1 µM, where these primers have the sequence:
BM1270: 5'-CCT GAG TCC ACG TCA TTA TTC-3'; SEQ ID No:5 and
BM179: 5'-TGA AGC TAT TTA TTC TTG TTAC-3'; SEQ ID No:6

Reactions were performed according to the following thermocycling conditions: initial incubation for 10 min. at 95°C, followed by 35 cycles of 30 sec. at 95°C, 30 sec. at 50°C, and 60 sec at 72°C. A strong product fragment (1100 bp) was purified and isolated using agarose gel electrophoresis, cloned, and sequenced. The resulting sequence could be translated into a polypeptide that exhibited excellent homology to known *L-LDH-*encoding genes (*e.g.,* Genbank accession #M22305).

The coding sequence for the *B. megaterium LDH*-encoding gene was operatively linked to a promoter from the phosphoglycerate kinase (*PGK*) gene and a transcriptional terminator from the *GAL10* gene, both from the yeast S. *cerevisiae.* Two oligonucleotide primers, Bmeg5' and Bmeg3', were designed based on this sequence to introduce restriction sites at the ends of the coding sequence of the gene:
Bmeg5': 5'-GCT CTA GAT GAA AAC ACA ATT TAC ACC-3; SEQ ID No:7 and
Bmeg3': 5'-ATGG ATC CTT ACA CAA AAG CTC TGT CGC-3'; SEQ ID No:8

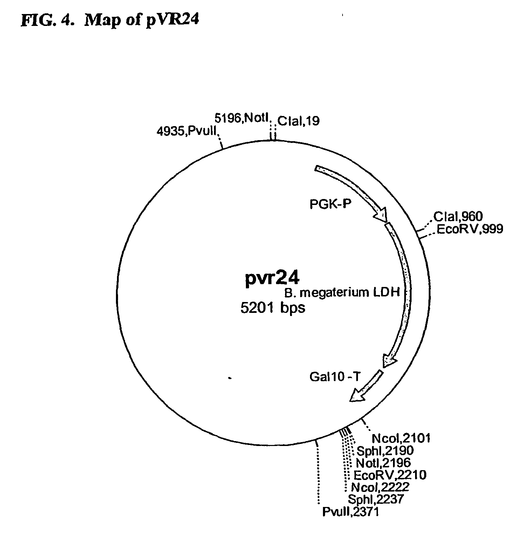

This amplification reaction was performed using dNTP and primer concentrations described above using *Pfu* Turbo polymerase (Stratagene) in the manufacturer's supplied buffer. Thermocycling was done with an initial incubation for 3 min. at 95°C, followed by 20 cycles of 30 sec. at 95°C, 30 sec. at 50°C, and 60 sec. at 72°C, followed by a final incubation for 9 min at 72°C. The product was digested with restriction enzymes *Xba*I and *BamHI* and ligated into the *Xba*I and *Bam*HI sites of plasmid pNC2. This ligation resulted in the *PGK* promoter and *GAL10* terminator becoming operably linked to the *B. megaterium LDH* coding sequence, identified as pVR24. (FIG. 4)

### Example 1C: Construction of pVR22 harboring the G418 resistance cassette operably linked to the PDC1 promoter and GAL10 terminator (S. cerevisiae).

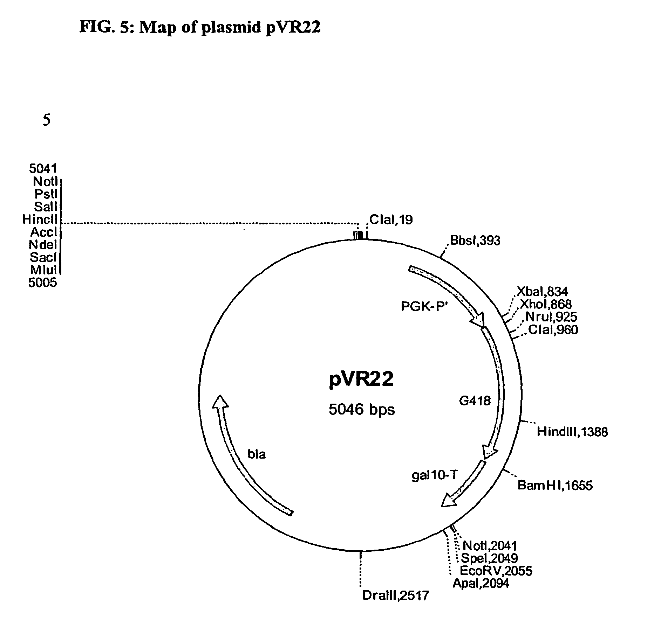

The G418 resistance marker was cloned under *S. cerevisiae PDC*1 promoter and the constructs were designated as pVR22 (shown in FIG. 5). The *S. cerevisiae GAL10* terminator was used in this plasmid as the terminator for G418 resistance gene. The G418 resistance gene was amplified by PCR using Pfu Turbo Polymerase (Stratagene) with primers 5'-GCT CTA GAT GAG CCA TAT TCA ACG GGA AAC (5' G fragment; SEQ ID No:9) and 5'-ATG GAT CCT TAG AAA AAC TCA TCG AGC ATC (3' G fragment; SEQ ID No: 10) and the plasmid, pPIC9K (Invitrogen, Carlsbad, CA), as the template. Thermocycling was done by initially incubating the reaction mixture for 5 min at 95°C, then by 35 cycles of 30 sec at 95°C, 30 sec at 49°C, 2 min at 72°C, followed by a final incubation for 10 min at 72°C. The PCR product was digested with *Bam*HI and *Xba*I and an 821 bp fragment was isolated and ligated to the 4303 bp *Bam*HI-*Xba*I fragment of pNC2. The resultant plasmid has the *PGK* promoter and *GAL10* terminator functionally linked to the G418 resistance gene was named pVR22, and is shown schematically in FIG. 5.

### Example 1D: Construction of the plasmid pNC7 for expression of Bacillus megaterium L-LDH gene on replicating plasmid, pKD1.

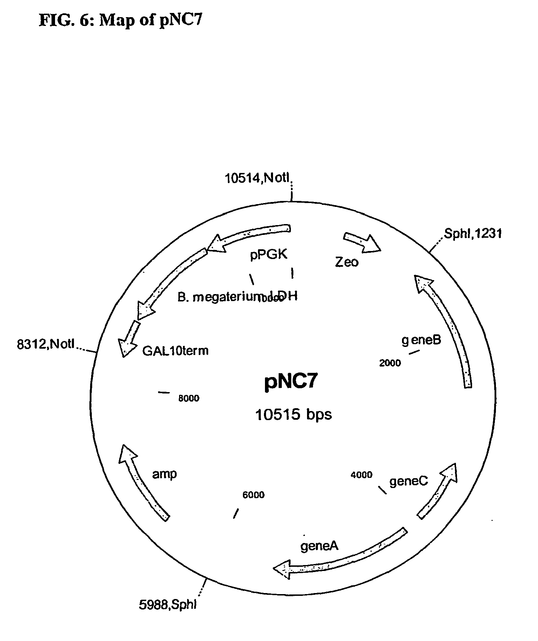

Plasmid pNC7 (FIG. 6) was constructed as follows. The expression cassette containing *B. megaterium-LDH* gene under transcriptional control of the *PGK* promoter and *GAL10* terminator (*S*. cerevisiae) was isolated from pVR24 as a *Not*I fragment, and ligated into plasmid pNC3 cut with *Not*I*,* to give pNC7. pNC3 was constructed by ligating the entire pKD 1 plasmid (see, Wesolowski-Louvel et al., Nonconventional Yeasts in Biotechnology; "Kluyveromyces lactis", pp. 139-201; K.Wolf ed.; Springer-Verlag, Berlin, 1996) linearized with *Sph1* into the unique *Sph1* site of *pTEF1*/*Zeo* (Invitrogen);

### Example 1E: Construction of Kluyveromyces marxianus strain NC39.

Plasmid pNC7 was transformed into wild-type *K.marxianus* (strain CD21) using standard methods to give a recombinant strain NC39 that had the *B. megaterium L-LDH* gene on a multi-copy pKD 1 plasmid.

### Example 1F: Construction of plasmids pSO28 and pSO29 for disruption of PDC1 disruption vectors

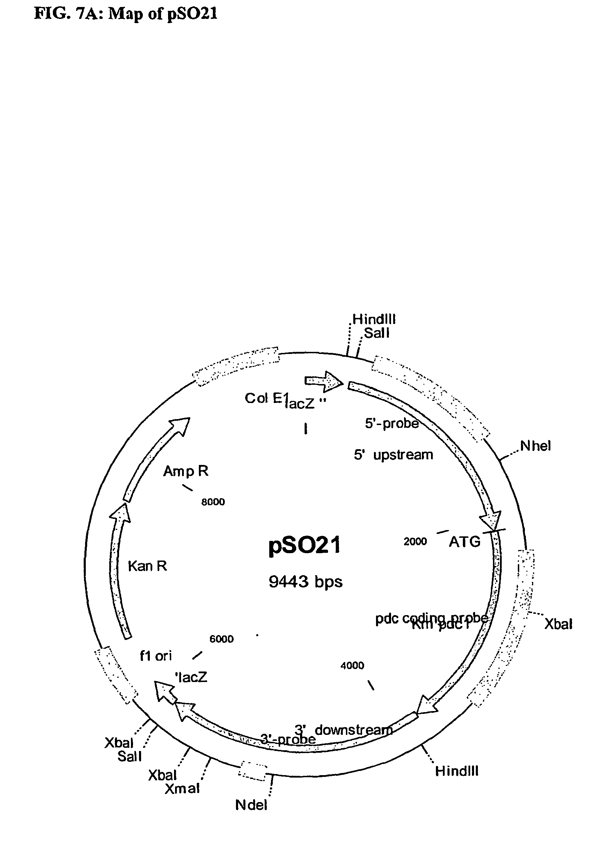

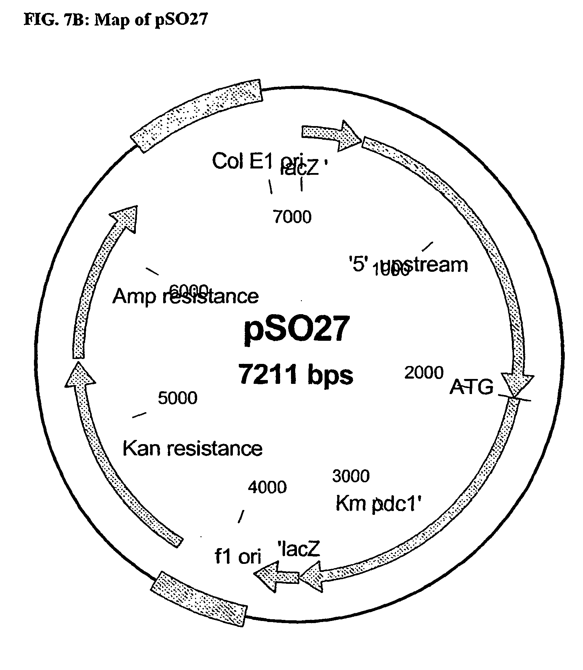

The plasmid pSO21 was constructed using the primers SO-M2 5'-CTT CCA GTC CAG CAG GTC GAC CAG- 3'; SEQ. ID No: 11, and SO-M1 5'-GTC CAG CAT GTC GAC CAG-3'; SEQ. ID.No:12. Genomic DNA from *K. marxianus* (strain CD21) was used as the template and the primers described above using conventional PCR methodologies a 5.5kb fragment was obtained that consisted of the *K. marxianus* PDC1 gene along with its promoter, terminator and large region upstream and downstream of the gene. The 5.5kb fragment was cloned into the pCRII (Invitrogen) plasmid to give plasmid pSO21 (FIG. 7a). The plasmid pSO27 (FIG. 7b) was synthesized by PCR amplification of the 3.3kb *PDC1* fragment (PDC1 gene, promoter) from pS021 using the primers SO-M4 5'-GAA CGA AAC GAA CTT CTC TC-3'; SEQ ID No: 13, and SO-M5 5'-CTT GGA ACT TCT TGT CAG TG-3'; SEQ ID No:14, using conventional PCR methodologies. The resulting fragment was purified and isolated by gel electrophoresis, and subsequently ligated into pCRII (Invitrogen) to give pS027.

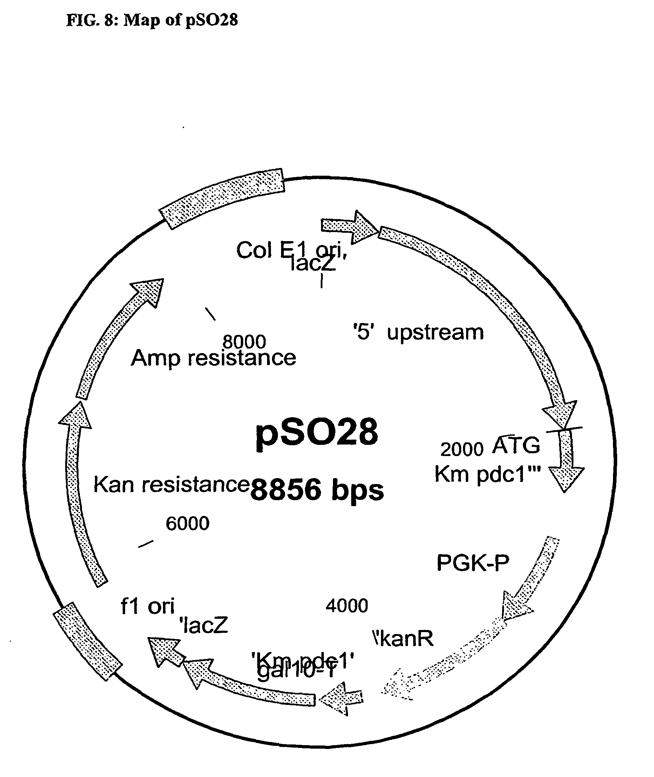

pSO28 (FIG. 8) was constructed by digesting pS027, harboring *PDC1,* with *Bbs*I to delete 417 bp from the *PDC1* coding region. The resulting nucleotide overhangs were filled in with *Pfu* DNA polymerase (Stratagene). This fragment (ca. 2.9kb) was ligated into a *Pfu*-blunt-ended *Not*I fragment from pVR22 (Example 1C) that contains the *S. cereuisiae PGK* promoter and the G418 resistance gene followed by the *S. cerevisiae GAL10* terminator.

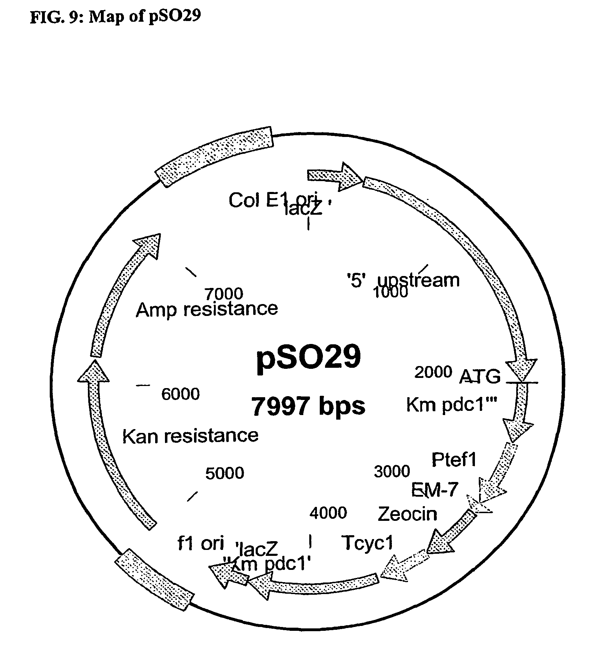

Similarly, pSO29 (FIG. 9) was constructed by digesting pSO27 with *Bbs*I to delete 417 bp from the *PDC1* coding region. The nucleotide overhangs were filled in with *Pfu* DNA polymerase (Stratagene). This fragment was ligated to a *Pfu*-blunted *Xh*oI/*Xb*aI fragment from p*TEF1*/Zeo (Invitrogen) that contains the *S. cerevisiae TEF1* promoter and the zeocin resistance gene followed by a *S. cerevisiae CYC1* terminator.

These constructs were made in order to design various integration cassettes useful for creating *K. marxianus* strains having a *pdc1* null genotype, with various integrated selection genes. The transformants are first screened to verify the insertion of the construct at the *PDC1* locus, and then screened for an inability to produce ethanol and inability to grow anaerobically. The preferred recombination event is a double cross over at *PDC1* on the genome. However, a single or a double recombination event can occur, or a DNA insertional mutagenesis event can occur.

### Example 1G: Transformation and selection of K. marxianus for pdc1 null genotype using Pdc- phenotype: Construction of strains CD 181, CD186, CD214, CD215, CD216, CD217, CD218, CD219, CD220, and CD221.

Strain NC39 was chosen as a transformation host because it contains plasmid pNC7 in the host as a replicating plasmid containing the *L-LDH* on the pKD 1 backbone the presence of which can increase the probability of a deletion at *PDC1* (*see,* Chen XJ, et al., Curr. Genet., 1989 Aug; 16(2):95-8).

NC39 was grown overnight in YPD with 100 µg/mL zeocin (Invitrogen) selection in order to retain the pNC7 plasmid, and the transformation was carried out according to the following yeast chemical transformation protocol. Cells were grown overnight in 5 mL YPD medium at 30°C, with shaking at 250 rpm. The culture was diluted with 50 mL of YPD, to a starting OD₆₀₀ of about 0.2. The culture was then grown at 30°C, with 250 rpm until the OD₆₀₀ was about 3.0. The cells were then harvested by centrifugation at 2500 rpm for 5 min, and resuspended in 50 mL sterile water. The cells were harvested by centrifugation at 2500 rpm for 5 min. The resuspension centrifugation was repeated once. Following that step, the cell pellet was resuspended in 1.0 mL of 10mM Tris, pH 7.5, 1mM EDTA, 20mM lithium acetate, pH 7.5. Into a microfuge tube was added: 4.0 µg of linear pSO28 DNA fragment cut with *Sac*I/*Apa*I containing the G418 selection marker and the flanking *PDC1* region, 25 µL of carrier DNA (Colette - 10mg/mL sand solicited), and 500 µL of the prepared NC39 cells were added and mixed by vortex. To this was added 3 mL of 40% PEG, 10mM Tris, pH7.5, 1mM EDTA, 20mM lithium acetate, pH 7.5 and mixed by vortex. This mixture was then incubated for 30 min at 30°C, with shaking at 250 rpm. After the incubation period, 350 µL DMSO was added and mixed by inversion of tubes. The cells were then heat shocked at 42°C for 15 minutes, followed by fast cooling on ice for about 3 min. The cells were pelleted by a 10-second spin at 14,000 rpm, and the supernatant removed. The cells were resuspended in 1mL YPD. The cells were allowed to recover by incubation for 4 hrs at 30°C, with shaking at 250 rpm. After the recovery period, the cells were pelleted and the supernatant removed. At this point the cells were resuspended in a final volume of about 500 µL of YPD. An aliquot (20 µL) of the cell suspension was plated on one selection plate and aliquots of 100 µL were plated on 6 other selection plates. The plated cells were allowed to incubate at 30°C until colony growth was observed.

The plates used for this transformation contained 300 µg/mL of selection agent (G418). Transformants that grew on these primary plates were then patch-plated to secondary plates with the same concentration of selection agent. Colonies growing on these secondary plates were screened by PCR, using conventional methods, for integration events at *PDC1* using a 3' primer downstream and outside of the integration fragment (SO454 5'-CCA TCC GAA GAA GTC GAT CTC-3'; SEQ ID No:15) and the 5' primer lying within the G418 resistance gene (SO285 5'-CTT GCC ATC CTA TGG AAC TGC-3'; SEQ ID No: 16). By this PCR analysis only 1 of 200 colonies was positive. Additional PCR analysis using conventional PCR methods and *PDC1* primers targeted to the deleted *BbsI* fragment from pSO27 (-SO2740 5'-GAA GGC TGG GAA TTG AGT GA-3'; SEQ ID No: 17 and -SO244 5'-GCT CCA TCT GAA ATC GAC AG- 3'; SEQ ID No: 18) was used to confirm the presence of intact wild-type *PDC1.*

The single positive transformant was isolated as a single colony, used to produce a glycerol stock, and identified as CD181. The strain was then cured of the pNC7 plasmid by culturing without selection pressure, followed with plating the cultured cells on YPD agar plates comprising G418 (300µg/mL). Four colonies were picked and tested for zeocin sensitivity and lack of L-lactic acid production. All four colonies exhibited zeocin sensitivity and had lost the ability to produce L-lactic acid. A single colony was chosen and used to produce a glycerol stock, and identified as CD 186.

In order create a *K. marxianus* strain with a PDC- phenotype, (no ethanol production and no growth under anaerobic conditions), another transformation of CD186 was necessary. CD186 was transformed, according to the above protocol, with 6.8 µg of linearized pSO29 DNA fragment, cut with *Nhe*I/*Not*I*,* containing the zeocin selection marker and the deletion in the *PDC1* region.

The resulting transformants were plated on YPD agar plates comprising 300 µg/mL zeocin. Transformants growing on these primary plates were patch-plated onto secondary plates with 300 µg/mL G418 and 300 µg/mL zeocin. Colonies growing on these secondary plates were screened for integration at the *PDC1* locus by PCR, that tested for the absence of the wild-type *PDC1,* using primers that are present on the deleted *Bbs*I fragment from pSO27, -SO2740 5'-GAA GGC TGG GAA TTG AGT GA-3'; SEQ ID No: 17, and SO2444 5'-GCT CCA TCT GAA ATC GAC AG-3'; SEQ ID No: 18.

Eight colonies were found to have deletions in *PDC1.* This was verified by PCR analysis that showed the expected deletion from the wild-type *PDC1,* and the PDC- phenotype (no ethanol production or anaerobic growth). Single colonies from these eight transformants were isolated, and entered into the Cargill Dow Culture collection. These eight *K. marxianus pdc1* null strains were named CD214, CD215, CD216, CD217, CD218, CD219, CD220, and CD221. CD215 was tested for production of ethanol by inoculating a colony into 50 mL YPD medium in a 250 shake-flask. The culture was incubated at 30°C with shaking at 70 rpm and HPLC analysis of the samples did not detect any ethanol.

### Example 1H: Construction of plasmid pPS1, functionally linking the hygromycin resistance gene to the PDC1 promoter and GAL10 terminator of S. cerevisiae.

A recombinant nucleic acid conferring hygromycin resistance to transformed yeast cells, permitting selection of yeast cell transformants comprising a recombinant nucleic acid construct encoding a protein useful for synthesis of an organic product, was prepared as follows. The hygromycin resistance marker (*E. coli hph*) was cloned under the transcriptional control of *Saccharomyces cerevisiae PDC1* promoter.

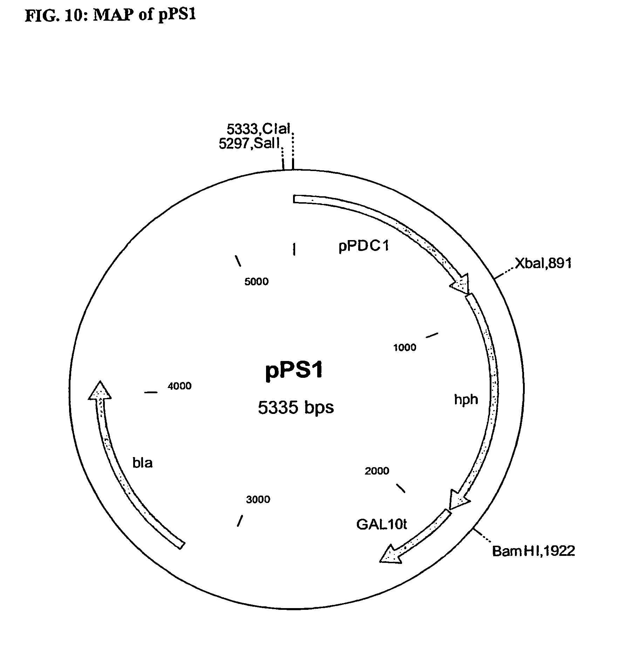

The *E. coli hph* gene that confers resistance to hygromycin B was PCR amplified using the primers 5'HYGXBA1 (5'-AAG CTC TAG ATG AAA AAG CCT GAA CTC AC-3'; SEQ ID No: 19) and 3'HYGBAMH1 (5'-CGC GGA TCC CTA TTC CTT TGC CCT CGG AC-3'; SEQ ID No:20) and the plasmid pRLMex30 (see, *e.g.,* Mach et al. 1994, Curr. Genet. 25, 567-570; Rajgarhia et al., U.S. Patent Application No: 10/154,460, filed May 23, 2002, incorporated by reference in its entirety) as the template. The *hph* gene can also be obtained by using the same primers with *E. coli* chromosomal DNA serving as template. Thermocycling was performed by 30 cycles of 1 min at 94°C, 1 min at 56°C, 3 min at 72°C, followed by a final incubation of 7 min at 72°C using Pfu Turbo DNA polymerase (Stratagene). The PCR product was electrophoretically separated and on a 0.8% agarose gel and the 1026 bp product isolated. The PCR product was digested with *Xba*I and *Bam**HI and ligated into the *Xba*I and *Bam*HI cut plasmid pNC4 to give the plasmid pPSl (see FIG. 10).

### Example 1I: Construction of pVR43 containing L. helveticus D-LDH

*D-LDH* was isolated from *Lactobacillus helveticus* as follows. *Lactobacillus helveticus* cells were obtained from the American Type Culture Collection (ATCC Accession # 10797) and grown under standard conditions. Genomic DNA was purified from these cells using the following protocol:
1. A single colony or 5 µL from a glycerol stock of lactic acid bacteria was used to inoculate 2x 50 mL sterile MRS broth in 250 mL sterile flasks. The culture was grown with agitation at 37 °C for 48 hours at 170 rpm.
2. The culture was transferred to 50 mL sterile blue-capped tubes and centrifuged at 3000 rpm for 10 mins. The cell pellet was resuspended in 50 mL of 12.5% w/v sucrose solution and centrifuged again at 3000 rpm for 10 mins. The pellets were resuspended and combined in 5 mL of 12.5% w/v sucrose in a 50 mL sterile blue-capped tubes from Falcon (Cat. No. 29050). To the cell suspension was added 5 mL of TES solution (TES is: 10 mM Tris (pH 8.0), 50 mM EDTA (pH 8.0), 50 mM NaCl, sterile filtered). Another 5 mL of 25% w/v sucrose solution was added and then mixed. Lysozyme powder (300 mg) was added to the suspension and vortexed to mix. Once thoroughly mixed, 25 µL of mutanolysin solution (stock conc. 2.2 mg/mL) was added to the mixture. The suspension was incubated overnight (~10-12 hrs.) at 37°C.
3. Following the overnight incubation, 2.5 mL of a 20% SDS solution and 168 µL of a Proteinase K solution (stock conc. 28 mg/1.4 mL) were added. The tube was mixed by inversion, but not shaken. The tube was incubated at 50°C for 1 hour. At this point the cell membrane matter appeared broken up and solution became translucent. Enough NaCl was added to obtain a 0.15 M concentration in the solution. The solution was mixed thoroughly by inversion.
4. The mixture was transferred to a 50 mL Oakridge tube (Beckman Instruments Inc., Palo Alto, CA) or split between two tubes and treated with an equal volume of phenol:chloroform:isoamyl alcohol (25:24: 1) solution. The mixture was agitated and centrifuged at 10,000 rpm for 10 min.
5. The aqueous supernatant was removed to a clean Oakridge tube, and an equal volume of chloroform was added. The solution was mixed by shaking and centrifuged at 5000 rpm for 10 min.
6. The supernatant was siphoned off and put into a fresh tube. To the supernatant was added 25 µL of RNAse (stock conc. 100 mg/mL), which was mixed by inversion. The tube was incubated at 37°C for 15 min. The DNAse free RNAse is added in,excess to quickly degrade the RNA.
7. Lastly, 2.5 volumes of EtOH was added to the mixture by careful dispensing along the sides of the tube. The EtOH layer was not mixed. The DNA that forms at the liquid interface was spooled using a glass pipette and washed in 70% EtOH. The DNA was air dried and resuspended in 10 mM Tris-HCl, pH 8.5 (elution buffer in most Qiagen kits) in a microfuge tube. The tube was incubated at 50°C until the DNA went into solution.

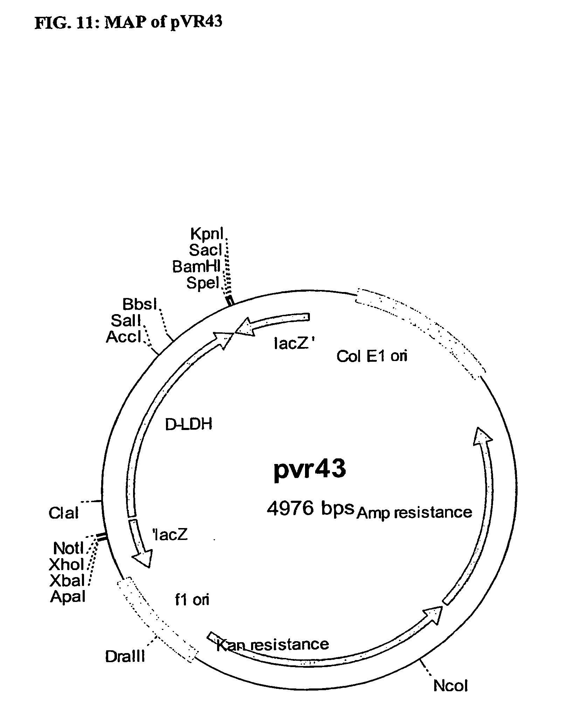

Primers were designed based on the available sequence in Genbank for *D-LDH* from *L. helveticus* (Genbank accession #U07604 or #X66723 (SEQ ID No. 43)). PCR amplification reactions were performed using Pfu Turbo polymerase (Stratagene, WI, USA). Each reaction contained *L. helveticus* genomic DNA at a concentration of 500ng, each of 4 dNTPs at a concentration of 0.2 mM, and each of the amplification primers VR150 5'-GGT TGG ATT TAT GAC AAA GGT TTT GCTT-3'; SEQ ID No. 21) and VR153 5'-AAT TAA AAC TTG TTC TTG TTC AAA GCA ACT-3'; SEQ ID No. 22) at 1 µM. Reactions were performed according to the following cycling conditions: an initial incubation for 10 min at 95°C, followed by 35 cycles of 30 sec at 95°C, 30 sec at 51°C, and 60 sec at 72°C. A strong product fragment of 1027 bp was gel purified using conventional procedures and TA cloned into the PCR-BluntII topo cloning vector (Invitrogen, Carlsbad, CA). The resulting plasmid was sequenced and named pVR43 (shown in FIG 11a). The resulting sequence could be translated into a polypeptide that exhibited excellent homology to known *L. helveticus* D-LDH-encoding gene sequences in Genbank (Accession # U07604 or #X66723).

### Example 1J: Construction of pVR47, containing L. helveticus D-LDH under the control of the S. cerevisiae PGK1 promoter and GAL10 terminator.

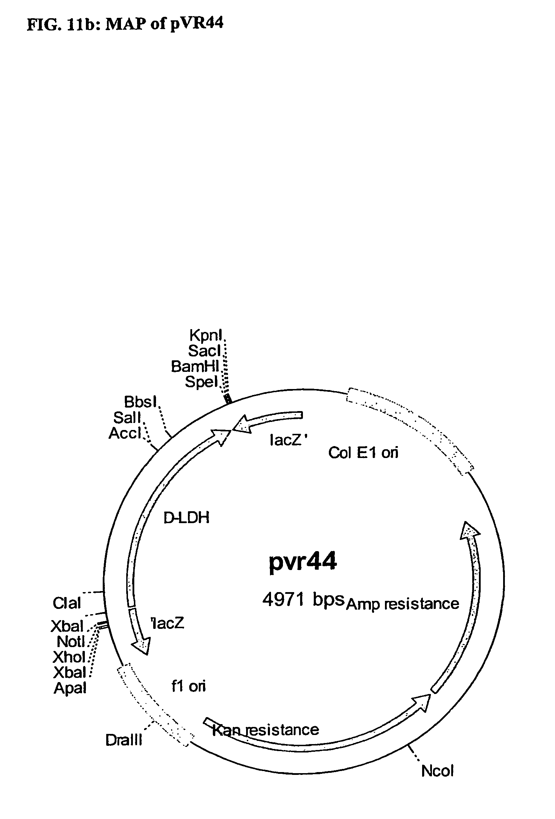

Primers were designed to introduce *Xb*aI and *Bam*HI sites at the 5' and 3' end of the *D-LDH* gene for cloning into pNC2. PCR amplification reactions were performed using Pfu Turbo polymerase (Stratagene, Wisconsin, USA). Each reaction contained pVR43 (harboring *L. helveticus D-LDH*) (5 ng), each of 4 dNTPs at a concentration of 0.2 mM, and each of the amplification primers VR165 5'-CGT CTA GAT TTA TGA CAA AGG TTT TGCT-3'; SEQ ID No. 23 and VR166 5'-GCG GAT CCT TAA AAC TTG TTC TTG TTC AA-3'; SEQ ID No. 24 at 1 µM. Reactions were performed according to the following cycling conditions: an initial incubation for 10 min at 95°C, followed by 35 cycles of 30 sec at 95°C, 30 sec at 55°C, and 60 sec at 72°C. A strong product fragment of 1035 bp was gel purified using conventional procedures and TA cloned using the PCR-BluntII topo cloning vector (Invitrogen, Carlsbad, CA). The resulting plasmid was sequenced and named pVR44 (see FIG. 11b). This sequence could be translated into a polypeptide that exhibited excellent homology to known *L. helveticus D-LDH* -encoding gene and had the *Xba*I and *Bam*HI sites at the 5' and 3' end of the *D-LDH* gene.

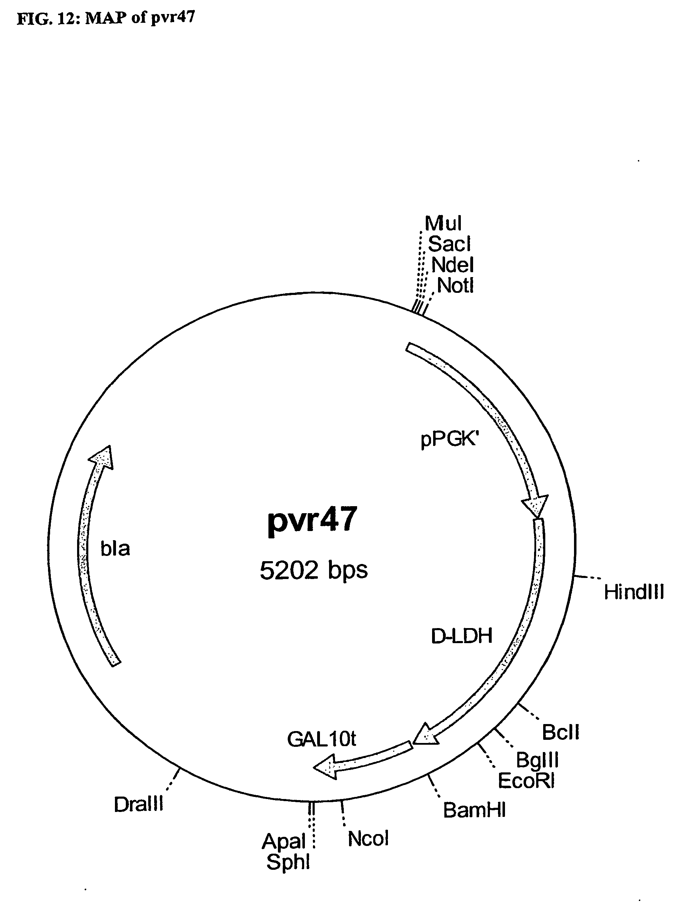

Plasmid pNC2 was digested with *Xba*I and *Bam*HI*.* The 5'-phosphate ends of the linearized pNC2 was dephosphorylated using shrimp alkaline phosphatase (Roche Diagnostics, USA) following the manufacturers protocol. pVR44 was also digested with *Xba*I and *Bam*HI and the 1027 bp *L. helveticus D-LDH* gene was isolated by gel electrophoresis (0.8% agarose gel). The two fragments were ligated and the resultant plasmid, named pVR47 (see FIG. 4), was sequenced. The pVR47 plasmid has the *S. cerevisiae PGK* promoter and *GAL10* terminator functionally linked (*i.e.,* transcriptionally-active in a yeast cell) to the *L. helveticus D-LDH* gene, as shown in FIG. 12 (SEQ ID NO 43).

### Example 1K: Construction of pVR48, containing L. helveticus D-LDH gene and the hygromycin resistance marker adjacent to each other

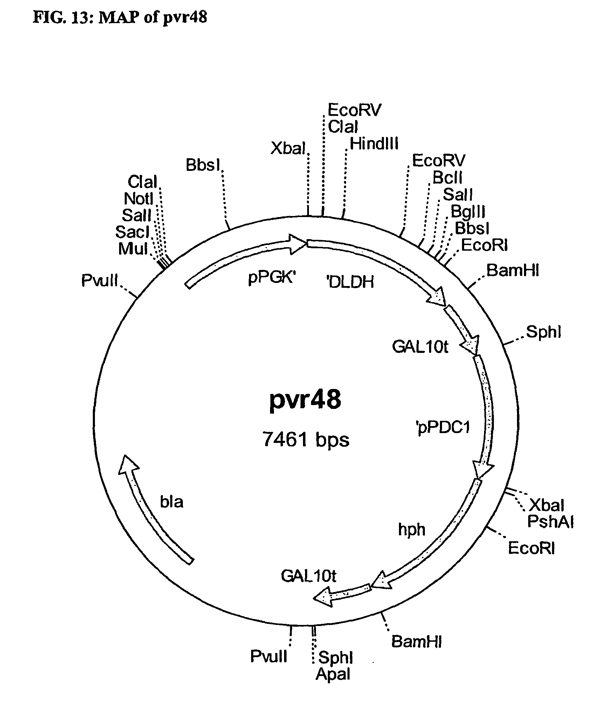

pPS1 was digested with *Sph*I and the 2.259 kbp fragment containing the hygromycin resistance gene expressed under the control of the S. *cerevisiae PDC1* promoter and *GAL10* terminator was isolated electrophoretically using a 0.8% agarose gel. pVR47 was digested with *Sph*I and the 5'-phosphate ends of the linearized plasmid was dephosphorylated using shrimp alkaline phosphatase (Roche Diagnostics, USA) following the manufacturer's protocol. The two fragments were ligated and the resultant plasmid was sequenced and named pVR48 (shown in FIG. 13). The plasmid contains the *L. helveticus D-LDH* gene and the hygromycin resistance marker cassettes adjacent to each other.

### Example 1L: Introduction of DNA encoding the L. helveticus D-LDH gene via random integration into the K. marxianus genome

A 4.54 kbp fragment containing the *L. helveticus D-LDH:HPH* resistance gene cassette was isolated from pVR48 by digesting the plasmid with *Sst*I and *Apa*I. The fragment was isolated by gel electrophoresis on a 0.8% agarose gel. This fragment was used to transform *pdc1* null mutant *Kluyveromyces marxianus* (CD215; see Examples 1F, 1G) using an electroporation protocol, described below:

A single colony of *K. marxianus* was used to inoculate 50 mL of YPD (comprising 10 g/L yeast extract, 20 g/L peptone, 20 g/L glucose and 2% agar) in a 250 mL baffled shake flask to an OD₆₀₀ of 0.1. The culture was grown 16 hrs at 30°C with 250 rpm to a final OD₆₀₀ of 10. The cells from 10 mL of culture were collected by centrifugation and washed one time with electroporation buffer (EB; 10 mM Tris-Cl, 270 mM sucrose, 1 mM MgCl₂, pH 7.5). The cells were resuspended in incubation buffer (IB; YPD + 25 mM DTT, 20mM HEPES, pH 8.0) and incubated at 30°C with 250 rpm for 30 min. The cells were then harvested by centrifugation and washed one time with EB. The cells were resuspended in 1 mL EB, and 400 µL of these cells were transferred to a 0.4 cm electroporation cuvette (BioRad; Hercules, CA).

2 µg of the 4.54 kbp *Sst*I/*Apa*I fragment from pVR48 was added to the cuvette and the cells were electroporated at 1.8 kV, 1000 Q, 25 µF. The cells were transferred to 1 mL YPD in a 50 mL screw cap Falcon tube and incubated at 30°C with 250 rpm for 4 hrs before selective plating on YPD containing 200 µg/mL hygromycin. The transformants were grown at 37°C for 3 days. The transformants that were resistant to hygromycin were restreaked onto fresh selective plates containing 200 µg/mL hygromycin.

### PCR analysis:

Verification of integration of the fragment into the genome of *K. marxianus* CD21 was made using two sets of PCR primers:
1. One set of primers was designed to be homologous to the *L. helveticus* D-LDH gene and a reverse primer homologous to the hygromycin resistance gene. The primers VR161 5'-AGT TGG TGT ATT TAA CAA GG-3'; SEQ ID No. 25 and VR142 5'-GTG ACA CCC TGT GCA CGG CGG GAG ATG-3'; SEQ ID No. 26 were designed to amplify a 1.748 kb product between *L. helveticus* D-LDH and the hygromycin resistance gene in strains that have the cassette and should not amplify any fragment in strains that do not have the cassette. Thermocycling was performed on transformant colonies using Taq DNA polymerase (Qiagen, USA) by initially incubating the reaction mixture for 2 min. at 94°C, followed by 35 cycles of 30 sec at 94°C, 30 sec at 43°C, 3 min at 72°C, followed by a final incubation of 7 min at 72°C.
2. A second set of primers was designed to be homologous to the *PGK* promoter region and a reverse primer homologous to the *L. helveticus* D-LDH. The primers VR173 5'-GCG ACG GCT CAC AGG TTT TG-3'; SEQ ID No. 27, and VR170 5'-CTT GTC TTG ACG TAA TAC ACG TGC AGC-3'; SEQ ID No. 28, were designed to amplify a 0.75 kb product between *PGK* promoter and the *L. helveticus* D-LDH gene in strains that have the cassette and should not amplify any fragment in strains that do not have the cassette. Thermocycling was performed on transformant colonies using Taq DNA polymerase (Qiagen, USA) by initially incubating the reaction mixture for 2 min at 94°C, followed by 35 cycles of 30 sec at 94°C, 30 sec at 55°C, 1 min at 72°C, followed by a final incubation of 7 min at 72°C. Of the analyzed transformants, nineteen yielded the expected PCR products.

### Enzymatic and GC verification of D-lactic acid:

The optical purity of the D-lactic acid produced by the transformants was determined using the D-lactic acid detection kit from Boehringer Mannheim (Roche Diagnostics, USA). The manufacturer's protocol was followed and indicated that six of the nineteen transformants produced 0% L-lactic acid.

Gas chromatographic (GC) analysis of the supernatant from the transformants indicated that the strains produced greater than 99% D-lactic acid.

### GC Analysis Method

The separation and quantification of "D" and "L" enantiomers of lactic acid is accomplished using a chiral gas chromatography method. The established method involves the base-catalyzed hydrolysis of samples in methanol, followed by acidification with sulfuric acid to catalyze esterification and subsequent extraction of methyl lactate enantiomers into methylene chloride. The organic layer is then analyzed by gas chromatography (GC) using a flame ionization detector (FID) [Hewlett Packard 6890 with split/splittless injector set in split mode, auto injection, and a FID detector]. Separation of the methyl lactate enantiomers is achieved using a chiral capillary column [30 meter x 0.25 mm i.d. β-Dex 325 capillary column (0.25 µm film thickness), Supelco.(#24308)]. The normalized relative percentages of "D" and "L" lactic acid are obtained from this method.

Samples for GC analysis are prepared by weighing an amount of sample (1.000 g) into a 20 mL glass vial, to which is added 4 mL of methanol. The vial is capped and 150 µL, of concentrated sulfuric acid is added slowly. The vial is placed at 65°C in Reati-therm heater/stirrer for 10 minutes. Deionized water is then added (5 mL) to the vial, followed by addition of 10 mL of methylene chloride. The vial is capped and vigorously shaken. Using a disposable plastic syringe fitted with tube-tipped filter, a portion of the bottom organic layer is removed and the remaining bottom organic layer is transferred to a 2 mL GC vial for injection into the GC.

### Example 1M: Production of D-lactic acid in YPD plus glucose medium in shake-flask cultures by K. marxianus harboring L. helveticus D-LDH gene integrated into the genome under buffered conditions

The six transformants that produced optically pure D-lactic acid were named CD554, CD555, CD556, CD557, CD558 and CD559. These strains were cultivated in 250 mL baffled shake flasks containing 50 mL YPD, supplemented with 100 g/L glucose, and were inoculated from YPD agar plates. The cultures were grown for 16 hours at 30°C with 250 rpm shaking to an OD₆₀₀ of 0.1. After determining that residual glucose remained in each flask and that the cells were in exponential growth phase, 4 g/L cell dry weight equivalents were harvested by centrifugation and resuspended in 250 mL baffled shake flasks containing 50 mL YPD supplemented with approximately 100 g/L glucose and 55 g/L CaCO₃. The cultures were placed at 30°C with 70 rpm. Samples were withdrawn at various time intervals and the cells were removed by filtration. Culture supernatant was analyzed for glucose, D-lactate, pyruvate and ethanol by HPLC (described below).

### HPLC Analysis Method

The HPLC methods utilized in the following experiments employ a Bio-Rad Fast Acid Analysis Column combined with a Rezex Fast Fruit Analysis Column, each containing styrenedivinyl benzene resin in the hydrogen form. The organic components are quantified with a Refractive Index detector combined with a UV detector at 220 nm, using isobutyric acid as an internal standard. Samples are prepared by weighing out a known amount of sample and diluting it in the presence of an internal standard. The resulting solution is injected into a HPLC system and quantified using known standards.

Under these culture conditions, CD554, CD555, CD556, CD557, CD558, CD559 strains produced greater than 99% D-lactic acid, with a 92 - 98 % yield based on glucose, a D-lactic acid titer ranging from 82 - 90 g/L, and a volumetric productivity greater than 2.2 g/L/hr at 30°C.

### Example 2A: Construction of strains with deletions of the PDC1 coding sequence and the L. helveticus D-LDH gene integrated at the PDC1 locus using a one-step replacement method.

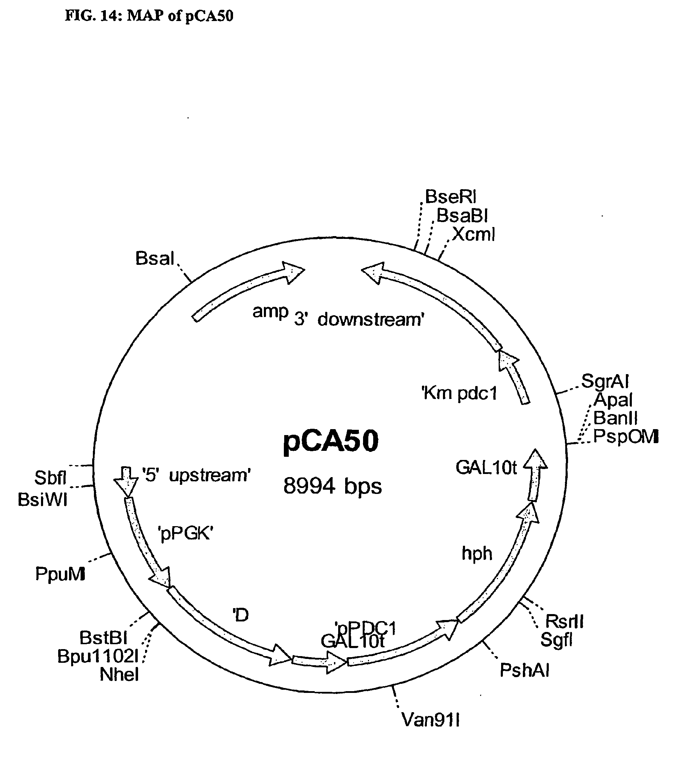

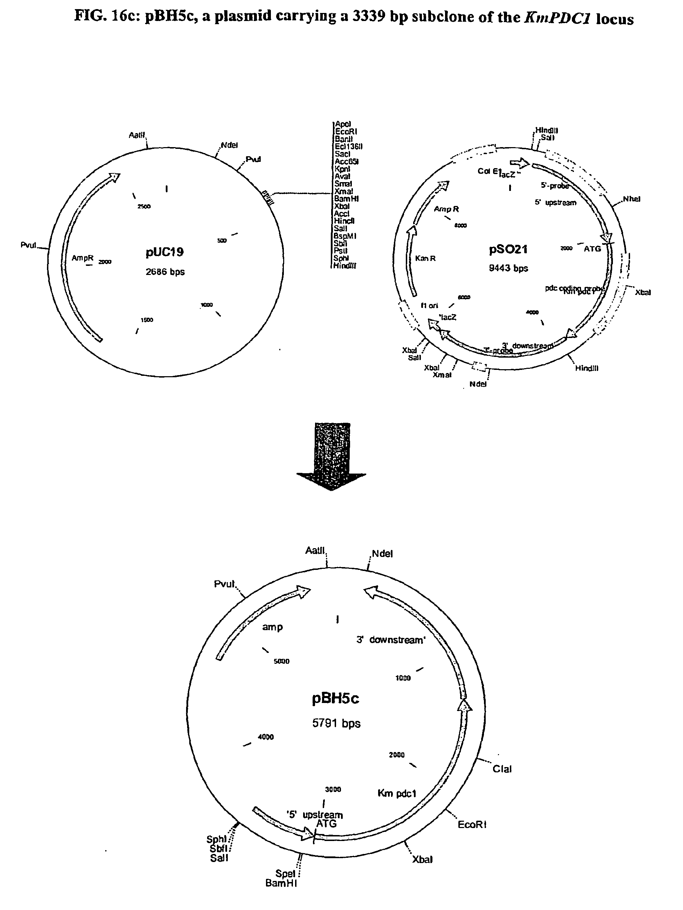

The plasmid pCA50 was constructed by ligating the 4.7kb *Ngo*MIV/*Psi*I fragment from plasmid pVR48 (Example 1K) into the backbone of plasmid pBH5c (described in Example 3B; FIG. 16c) cut with MscI and SgrAI. The pVR48 fragment contained the *L. helveticus D-LDH* gene driven by the *S. cerevisiae PGK* promoter, followed by the *S*. *cerevisiae GAL10* transcription termination sequence. This expression cassette is adjacent to the hygromycin resistance gene *(hph),* driven by the *S. cerevisiae PDC1* promoter, followed by the *GAL10* terminator *(S. cerevisiae).* The pVR48 fragment was ligated into the pBH5c backbone, generating the plasmid pCA50, wherein the *D-LDH:hph* construct is flanked by the sequences immediately upstream and downstream of the *K. marxianus PDC1* locus (FIG. 14).

The plasmid pCA50 was digested with *Sbf*I and *Bse*RI to generate a 6272bp fragment. 2.5 µg of this fragment was isolated and purified by gel electrophoresis to be used for transformation. Wild-type *K*. *marxianus* (CD21) was transformed with the purified fragment using the electroporation protocol disclosed in Example 1M. Transformed cells were plated on YPD selection plates containing 150 µg/mL hygromycin to select for cells that contain either a random integration of the pCA50 fragment into the genome, or a homologous replacement of the wild-type *PDC1* locus by the fragment. Colonies were isolated after 3 days growth at 30°C and streaked onto fresh YPD with 150 µg/mL hygromycin to confirm the phenotype.

The colonies were assayed by PCR in order to identify those that contained the homologous replacement of the wild-type *PDC1* locus by the pCA50 fragment. Cells were resuspended in the PCR reaction in order to provide template DNA. The 5' primer oCA85 5'-GGA CCG ATG GCT GTG TAG AA-3'; SEQ ID No. 29, was designed to amplify the 3' end of the hph gene. The 3' primer oCA80 5'-TCG CTT ACC TCG GTA CAG AA-3'; SEQ ID No. 30, was designed to amplify the sequence downstream of the *K. marxianus PDC1* locus, which is not contained in the pCA50 construct. The amplification assay used as a probe for the target sequence employed *Taq* polymerase (Qiagen) and the following PCR reaction conditions: initial melting step at 95°C for 10 min, followed by 40 amplification cycles of 30 sec at 95°C, 30 sec at 55°C, and 3 min at 72°C, followed by 10 min at 72°C.

Transformed cells having a homologous replacement event generated a 2.5 kb PCR product. Random integration events will not generate a PCR product with the oCA85 and oCA80 primers. Strains CD597 through CD601 were positive for this 2.5 kb PCR product.

### Example 2B: Production of D-lactic acid in complex CaCO₃ buffered media in microaerobic shake flask cultures of K. marxianus with a single copy of D-LDH integrated into the PDC1 locus using a one-step replacement method.

The *pdc1Δ::Lh-D-LDH* strain CD587 (**from Example 3E**) which is the result of a two-step replacement event was compared to the *pdc1Δ:.Lh-D-LDH* strains CD597, CD599, and CD600, generated by a one-step replacement event (from Example 2A) to determine if these strains produced similar lactic acid titers independent of the method of generation.

Biomass was generated in baffled shake flasks by growth overnight at 37°C at 225 rpm agitation in YPD+100g/L glucose and +50 g/L CaCO₃. Production flasks were inoculated with 2 g/L cell dry weight from the overnight biomass shake flasks. Production conditions were YPD media +100 g/L glucose and +50 g/L CaCO₃ with shaking at 70 rpm in baffled shake flasks at 35°C. Samples were taking at various time points: biomass and CaCO₃ were removed by filtration and filtrates were analyzed for glucose, lactate, EtOH and pyruvate by HPLC (Example 1M)

After 54 hours, CD587, CD589, and CD590 had consumed 85-88 g/L glucose, producing 81 g/L lactate and 2.5 g/L pyruvate. These lactate titers represent a 92-95% lactate yield on glucose and a volumetric productivity greater than 1.5 g/L hr during the microaerobic. These results show that the *D-LDH* transformants can produce D-lactic-acid. Enzymatic analysis of the lactic acid produced by these strains showed that the lactic acid in greater than 99% enantiomerically pure D-lactic acid.

These results show that the *D-LDH* transformants generated using a one-step gene replacement strategy (CD597, CD599 and CD600) can produce D-lactic acid similarly to the two-step replacement strain, CD587.

### Example 3A: Construction of the pVR29 plasmid having the G418 resistance gene functionally linked to the S. cererrisiae PGK promoter and GAL10 terminator (pVR29).

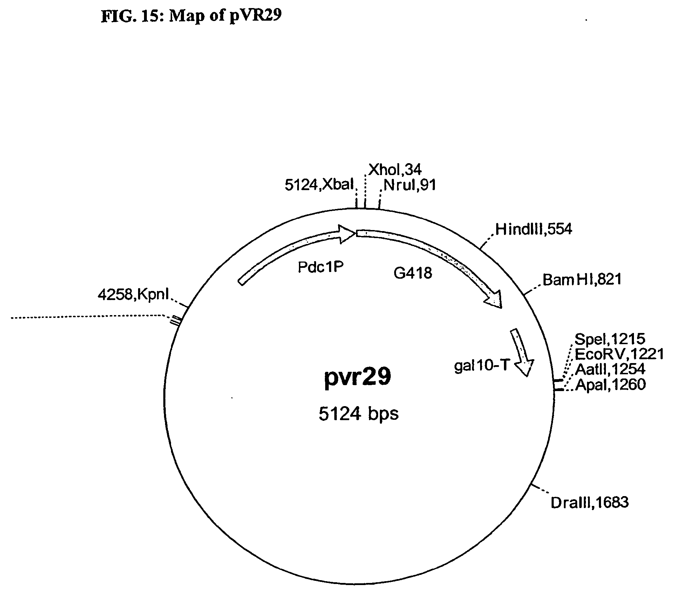

The G418 resistance marker (pVR22; Example 1C) was cloned into pNC2 (Example 1A) and the construct was designated pVR29 (FIG 15). The *S. cerevisiae PGK* promoter and the *S*. *cerevisiae GAL10* terminator was used to express the G418 resistance gene. The G418 resistance gene was amplified by PCR using the Pfu Polymerase (Stratagene, Madison, WI) with primers 5' - GCT CTA GAT GAG CCA TAT TCA ACG GGA AAC (5'G fragment; SEQ. ID NO. 9) and 5' - ATG GAT CCT TAG AAA AAC TCA TCG AGC ATC (3' G fragment; SEQ. ID NO. 10) and the plasmid pVR22 (Example 1C) as the template. Alternately plasmid pPIC9K (Invitrogen, Carlsbad, CA) can also be used as a template. Thermocycling was performed by initially incubating the reaction mixture for 5 min at 95°C, followed by 35 cycles of 30 sec at 95°C, 30 sec at 49°C, and 2 min at 72°C, followed by a final incubation for 10 min at 72°C. The PCR product was digested with BamHI and *Xba*I and a 821 bp fragment was isolated and ligated to the 4303 bp BamHI-XbaI fragment of pNC2. The resulting plasmid, pVR29 (FIG. 15) contained the *PGK* promoter and *GAL10* terminator operably linked (*i.e.*, transcriptionally-active in a yeast cell) to the G418 resistance gene.

### Example 3B: Plasmid for the targeted integration of DNA into the PDC1 locus of K. marxianus

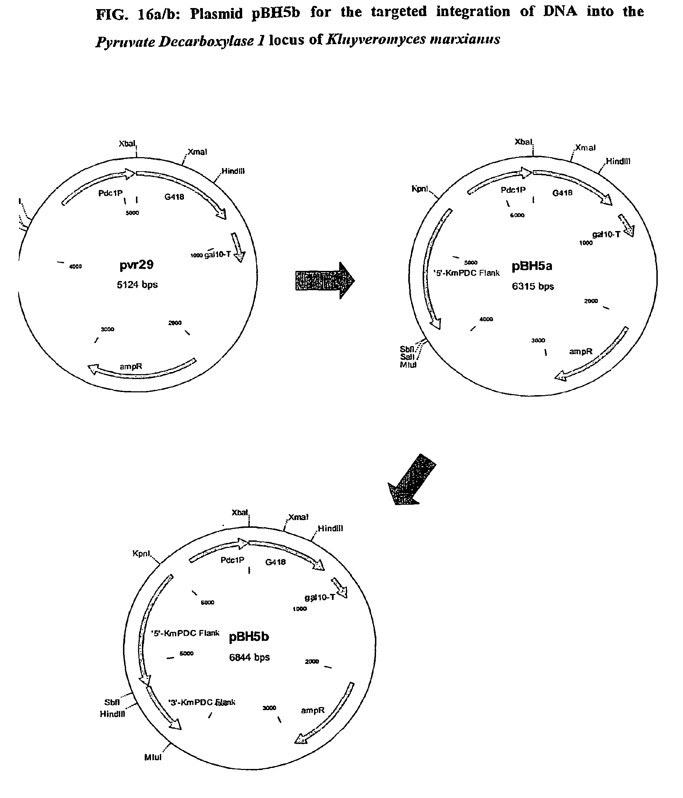

The DNA sequence flanking the *PDC1* gene of *K. marxianus* was cloned into the plasmid pVR29 (Example 3A, Fig. 15) to generate a recombinant nucleic acid for directed DNA integration at the *PDC1* locus. The resulting construct was designated pBH5a FIG. 16a) pBH5a comprises an *Sbf*I restriction site that allows cloned genes to be operatively linked to the *PDC1* promoter.

A 1254 bp fragment of DNA immediately upstream of *PDC1* was amplified by PCR with the primers 5'-CAA GAA GGT ACC CCT CTC TAA ACT TGA ACA-3' (5'-Flank 5'; SEQ ID No. 31) and 5'-GTA ATT CCT GCA GGT GCA ATT ATT TGG TTT GG-3' (5'-Flank 3'; SEQ ID No. 32) and the plasmid pSO21 (FIG. 7B, Example 1F) as the template. Thermocycling was done by initially incubating the reaction mixture for 2 min at 94°C, then by 35 cycles of 30 sec at 94°C, 30 sec at 55°C, and 1.5 min at 72°C, followed by a final incubation of 7 min at 72°C. The 1254 bp PCR product was electrophoretically separated on a 0.8% agarose gel and isolated. The PCR product and pVR29 (FIG 15a) were both digested with *Kpn*I and *Sbf*I. The digested PCR product was ligated to the 5067 bp pVR29 to give the 6315 bp pBH5a (see FIG. 16A). The resulting plasmid contains a G418 resistance gene operatively linked to the *S. cerevisiae PDC1* promoter and *GAL10* terminator and a 1240 bp fragment of DNA homologous to DNA immediately upstream of the *PDC1* gene.

A 535 bp fragment of DNA immediately downstream of *PDC1* was PCR amplified with primers 5'-CCA AGC CCT GCA GGA GAG GGA GAG GAT AAA GA-3' (3'-FIank 5'; SEQ ID No. 33) and 5'-CTC GTA ACG CGT GTA CAA GTT GTG GAA CAA-3' (3'-Flank 3': SEQ ID No. 34) using the plasmid pS021 (FIG. 7A) as the template. Thermocycling was done by initially incubating the reaction mixture for 2 min. at 94°C, then amplified by 35 cycles of 30 sec at 94°C, 30 sec at 55°C, and 45 sec at 72°C, followed by a final incubation of 4 min at 72°C. The PCR product was separated electrophoretically on a 0.8% agarose gel and the 535 bp product isolated. The PCR product was digested with *Sbf*I and *Mlu*I*.* The 529 bp fragment was ligated with the *Sbf*I*-Mlu*I fragment of pBH5a to give the plasmid pBH5b. pBH5b contains 1.2kb of DNA immediately upstream, and 0.5kb of DNA immediately downstream, of *PDC1* with a unique *Sbf*I site located between the flanking *pdc1* sequences, also including a selectable G418 resistance marker operatively linked to the *S*. *cerevisiae PDC1* promoter and *GAL10* terminator, and is shown schematically in FIG. 16b.

A portion of the *PDC1 (K. marxianus)* locus was also subcloned into the commercial plasmid pUC19 (Invitrogen) to generate pBH5c, as follows. Plasmid pSO21 was digested with *Nhe* I and *Nde* I and the 3339 bp DNA fragment containing 486 bp of DNA immediately upstream of *PDC1,* the complete sequence of *PDC1,* and 1157 bp of DNA immediately downstream of *PDC1* was isolated following electrophoretic separation on a 0.8% agarose gel. pUC 19 was digested with *Xba*I and *Nde*I and the 2452 bp DNA fragment was isolated following electrophoretic separation on a 0.8% agarose gel. The digested pUC 19 and the *PDC1* locus were ligated together to give the 5791 bp pBH5c, and shown schematically in FIG. 16c.

### Example 3C: Plasmid for the targeted integration of the L. helveticus D-LDH gene into the PDC1 locus of K. marxianus

The *D-LDH* gene from pVR48 (Example 1K) was cloned into the *Sbf*I site of pBH5b to create a recombinant nucleic acid capable of integrating the *D-LDH* into the *K. marxianus PDC1* locus and expressing *D-LDH* under control of the endogenous *PDC1* promoter and terminator.

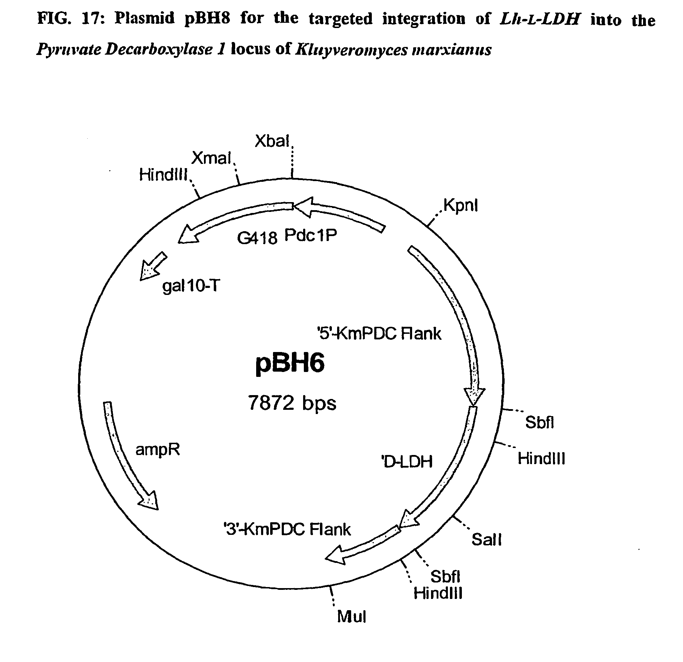

The *D-LDH* gene was PCR amplified using the primers 5'-TTT TTA CCT GCA GGC TAG ATT TAT GAC AAA GG-3' (*Lh-D-LDH* 5'; SEQ ID No. 35) and 5'-TCT ACC CCT GCA GGA AAA CTT GTT CTT GTT CA-3' *(Lh-D-LDH 3';* SEQ ID No. 36) using pVR47 (Example 1J) as a template. Thermocycling was performed by 30 cycles of 30 sec at 94°C, 30 sec at 55°C, 1.5 min at 72°C, followed by a final incubation for 4 min at 72°C using the Failsafe PCR System (Epicentre, Madison, WI). The PCR product was electrophoretically separated on a 0.8% agarose gel and the 1028 bp product was isolated. The PCR product was digested with *Sbf*I and ligated to the 6844 bp *Sbf*I-digested pBH5b to give the 7872 bp plasmid pBH6. pBH6 contains the *D-LDH* gene operatively linked to the *PDC1* promoter and terminator, along with a G418 resistance marker operatively linked to the *S. cereuisiae PDC1* promoter and *GAL10* terminator, shown schematically in FIG. 17.

### Example 3D: Integration of pBH6 into the PDC1 locus, creating the K. marxianus strain CD588

The integration plasmid pBH6, containing *D-LDH* was transformed into wild type *K. marxianus* (CD21). The entire plasmid was integrated into the *PDC1* locus via an initial single homologous recombination between the flanking *PDC1* DNA immediately upstream of the *D-LDH* gene of pBH6 and the DNA immediately upstream of the *PDC1* gene on the chromosome. The resulting strain contains the wild type copy of *PDC1* as well as a single copy pBH6 integrated at the *PDC1* locus.

CD21 was used to inoculate 50 mL of YPD, supplemented with 100 g/L glucose in a 250 mL baffled shake flask to an OD₆₀₀ of 0.1. The culture was grown for 16 hrs at 30°C with 250 rpm to a final OD₆₀₀ of 12. The cells from 10 mL of culture were collected by centrifugation and washed once with electroporation buffer (EB; 10 mM Tris-Cl, 270 mM sucrose, 1 mM MgCl₂, pH 7.5). The cells were resuspended in incubation buffer (YPD + 25 mM DTT, 20mM HEPES, pH 8.0) and incubated at 30°C with 250 rpm for 30 min. The cells were harvested by centrifugation and washed once with EB. The cells were resuspended in 1 mL EB, and 400 µL of this suspension was transferred to a 0.4 cm electroporation cuvette. 12 µg of uncut pBH6 in a total volume of 50 µL was added to the cuvette and the cells were electroporated at 1.8 kV, 1000 Ω, 25 µF. The cells were transferred into 1 mL YPD in a 50 mL screw cap Falcon tube and incubated at 30°C with 250 rpm shaking for 4 hrs before selective plating on YPD containing 300 µg/mL G418. The transformants were grown at 37°C for 2 days. Transformants that grew were streaked onto fresh selection plates.

Verification of proper integration of pBH6 into the *PDC1* locus via a single homologous recombination between the flanking *PDC1* DNA immediately upstream of the *D-LDH* gene of pBH6 and the DNA immediately upstream of the *PDC1* gene on the *K. marxianus* chromosome was evaluated using primers 5'-AAG CAC CAA GGC CTT CAA CAG-3' *(PDC1* Chromosome 5'; SEQ ID No. 37) and 5'-CTT GTC TTG ACG TAA TAC ACG TGC AGC-3'(*D-LDH* 3'; SEQ ID No. 38) designed to amplify a 2.7 kb product between chromosomal DNA upstream of the *PDC1* gene and outside of the homology incorporated in pBH6, and the *D-LDH* gene. Thermocycling was performed by incubating the reaction mixture for 2 min at 94°C, followed by 35 cycles of 30 sec at 94°C, 30 sec at 55°C, and 3 min at 72°C, followed by a final incubation of 7 min at 72°C. Seven of the ten transformants analyzed gave the expected 2.7 kb PCR product. Two products resulted from PCR analysis, using primers designed to amplify the *PDC1* locus 5'-CGC AAA GAA AAG CTC CAC ACC-3' (PDC1 5'; SEQ ID No. 39) and 5'-CCC ATA CGC TTA TAA TCC CCC-3' (PDC 1 3'; SEQ ID No. 40), a 1.7 kb band corresponding to *PDC1,* and a 1.0 kb band corresponding to *D-LDH.* Thermocycling was performed by incubating the reaction mixture for 2 min. at 94°C, followed by 35 amplification cycles of 30 sec at 94°C, 30 sec. at 55°C, 2 min. at 72°C, and by a final incubation of 5 min at 72°C. Southern blot analysis using a probe designed to detect the G418 resistance gene was used for further verification of single copy integration event. One of the seven transformants analyzed showed the proper banding pattern consistent with the integration of one copy of pBH6. The strain with one copy of pBH6 located at *PDC1* locus, as verified by PCR and Southern analysis, was designated CD588.

These results demonstrate that targeted integration of the transformed pBH6 DNA into the *PDC1* locus of wild-type *K. marxianus* CD21 has occurred through a single homologous recombination between the *PDC1* promoter sequences. These results also confirm that pBH6 is present as a single copy in strain CD588.

### Example 3E: Loss of pBH6 plasmid backbone and the PDC1 gene from CD588, generating K. marxianus strains CD587, CD589 and CD590

CD588 was propagated on non-selective media for several rounds of growth to encourage a second homologous recombination between the *PDCI* terminator sequences of the integrated pBH6 and the native *PDC*1 terminator. A second homologous recombination event in the terminator sequences resulted in a strain where the *PDC1* gene was replaced by the D-*LDH* gene. This strain is free of the G418 resistance marker gene due to loss of the G418 gene, along with the *PDC1* gene, occurring as a result of the second recombination event. The resultant strain wherein *PDC1* is replaced by *D-LDH* exhibited a G418-sensitive phenotype along with a lack of anaerobic growth.

CD588 was plated on YPD agar plates and grown overnight at 37°C. A swab of colonies was transferred to fresh YPD agar plates. This was repeated four times. Following the fifth round of non-selective growth on YPD agar plates, 6x 250 mL baffled shake flasks containing 50 mL YPD supplemented with 100 g/L glucose and 42 g/L CaCO₃ were inoculated to an OD₆₀₀ of 0.1 with CD588 cells from the fifth round of non-selective plating. The shake flasks were grown at 30°C with 250 rpm for 16 hrs. The cultures were then diluted and used to plate for single colonies on YPD agar plates. In addition, a swab of colonies from the fifth round of non-selective growth plates was suspended in 1 mL PBS (phosphate-buffered saline) and subsequently diluted and used to plate for single colonies on YPD agar plates. Single colonies resulting from this round of plating, were plated on YPD agar and placed in an anaerobic chamber. Following growth for 2 days at 37°C, the anaerobic chamber was opened and colonies that grew anaerobically were marked. The plates were incubated an additional day at 37°C. Colonies that grew aerobically but not anaerobically were transferred to triplicate plates for screening. The plates used for these screening conditions were YPD plates (aerobic), YPD plates (anaerobic), and YPD plates supplemented with 300 µg/mL G418. Three transformants were identified with the desired phenotype having G418 sensitivity and a lack of anaerobic growth. These transformants were designated CD587, CD589, and CD590.

Confirmation of the replacement of *PDC1* with *D-LDH* was performed using primers 5'-CGC AAA GAA AAG CTC CAC ACC-3' *(PDC1* 5'; SEQ ID No. 39) and 5'-CCC ATA CGC TTA TAA TCC CCC-3' *(PDC1* 3'; SEQ ID No. 40), designed to amplify the *PDC1* locus, using chromosomal DNA from CD587, CD589, and CD590 as templates. Thermocycling was performed by initially incubating the reaction mixture for 2 min at 94°C, followed by 35 cycles of 30 sec at 94°C, 30 sec at 55°C, 2 min at 72°C, and by a final incubation of 5 min at 72°C. Chromosomal DNA from all three strains yielded a single 1.0 kb PCR product corresponding to *D-LDH.* Southern blot analysis using a probe designed to hybridize to the G418 gene indicated the absence of the G418 gene. Further, Southern analysis with a probe for the *PDC1* coding region did not show any bands. Probes designed to hybridize to regions immediately upstream and downstream of *PDC1* gave bands with sizes consistent with the replacement of *PDC1* with *D-LDH.*

These results demonstrate that the *PDC1* gene, along with the pBH6 plasmid backbone containing the G418 resistance marker operatively linked to the *S. cerevisiae PDC1* promoter and *GAL10* terminator, were lost from the chromosome of CD588 through a second homologous recombination event that occurred between the two *pdc1* terminators present at the *PDC1* locus of CD588. This second recombination event provides a strain where the *PDC1* gene has been exactly replaced by the *D-LDH* gene flanked by *Sbf*I sites.

### Example 3F: Production of D-lactic acid in complex CaCO₃ buffered media in microaerobic shake flask cultures of K. marxianus with a single copy of D-LDH integrated into the PDC1 locus

The Pdc+ *Lh-D-LDH* strain CD588 and the Pdc- *Lh-D-LDH* strains CD587, CD589, and CD590 were cultivated in 250 mL baffled shake flasks containing 50 mL YPD supplemented with 100 g/L glucose and 50 g/L CaCO₃, following inoculation to an OD₆₀₀ of 0.1 from YPD agar plates, for 16 hours at 30°C with 250 rpm. After ensuring that residual glucose remained in each flask and that the cells were in exponential growth phase, 4 g/L cell dry weight equivalents were harvested by centrifugation and resuspended in 250 mL baffled shake flasks containing 50 mL YPD supplemented with 100 g/L glucose and 50 g/L CaCO₃. The cultures were placed at 30°C with 70 rpm. Samples were withdrawn at various time intervals and the cells were removed by filtration. Culture supernatant was analyzed for glucose, lactate, pyruvate and ethanol by HPLC (described above Example 1M).

After 23 hours, the Pdc+ *Lh-D-LDH* strain CD588 had consumed 127 g/L glucose, and produced 33 g/L lactate, 37 g/L ethanol, and 0.2 g/L pyruvate. The Pdc- *Lh-D-LDH* strains CD587, CD589, and CD590 had consumed 40-43 g/L glucose, producing 36-39 g/L lactate and 1.0-1.3 g/L pyruvate.

After 54 hours, CD587, CD589, and CD590 had consumed 85-88 g/L glucose, and produced 81 g/L lactate and 2.5 g/L pyruvate. These lactate titers represent a 92-95% lactate yield on glucose and a volumetric productivity greater than 1.5 g/L/hr during the microaerobic production phase. After 54 hours, an insoluble calcium lactate precipitate formed ("caked") due to the high levels of D-lactate produced in the cultures, preventing further analysis. Enzymatic and GC analysis, according to Example 1L, showed that more than 99% of the lactate produced was D-lactate. No ethanol was detected in cultures of CD587, CD589, and CD590, consistent with the replacement of *PDC1* by *LDH.*

These results show that the *D-LDH* transformants can produce D-lactic acid. Lactic acid production with a functional *PDC1* resulted in the approximate equal production of lactic acid and ethanol, demonstrating that the *D-LDH* can compete with the native *PDC1* for pyruvate. By replacing the *PDC1* with *D-LDH* from *L. helveticus,* the lactic acid titer increased two-fold, resulting in high titers and yields that approach the theoretical maximum. Further, these strains did not produce ethanol, demonstrating the replacement of *PDC1* with *D-LDH.* Enzymatic analysis of the lactic acid produced by these strains showed that the lactic acid in greater than 99% enantiomerically pure D-lactic acid.

### Example 3G: Production of D-lactic acid at high temperatures in complex CaCO₃ buffered media in microaerobic shake flask cultures of K. marxianus strains

Wild type *K. marxianus* CD21, the PDC+ *Lh D-LDH* strain CD588, and the Pdc- *Lh-D-LDH* strains CD558 and CD587 were inoculated to an OD₆₀₀ of 0.1 from YPD agar plates and were grown for 16 hours at 33°C with 250 rpm, in 250 mL baffled shake flasks containing 50 mL YPD supplemented with 100 g/L glucose and 50 g/L CaCO₃. After determining that residual glucose remained in each flask and that the cells were consequently in exponential growth phase, 4 g/L cell dry weight equivalents were harvested by centrifugation and resuspended in 250 mL baffled shake flasks containing 50 mL YPD supplemented with 100 g/L glucose and 50 g/L CaCO₃. The cultures were grown at 37°C, 42°C, or 50°C with 70 rpm. Samples were withdrawn at various time intervals and the cells were removed by filtration. Culture supernatant was analyzed for glucose, lactate, pyruvate, acetate, glycerol and ethanol by HPLC methods (From Example 1M).

After 23 hours, the PDC+ *Lh-D-LDH* strain CD588, fermenting at 37°C, had consumed 115.5 g/L glucose and produced 16.2 g/L lactate, 37.6 g/L ethanol, 7.6 g/L glycerol, 2.6 g/L acetate, and 0.2 g/L pyruvate. After 47 hours at 37°C, the PDC- *Lh-D-LDH* strains CD558 and CD587 had consumed 99-105 g/L glucose and produced 90-99 g/L lactate, 2.7-3.5 g/L pyruvate and 1.2-1.4 g/L acetate. In contrast, wild-type strain CD21 consumed 115.5 g/L glucose and produced principally ethanol (43.5 g/L) and a small amount of lactate (1.7 g/L).

After 23 hours, the PDC+ *Lh-D-LDH* strain CD588, fermenting at 42°C, had consumed 115.5 g/L glucose and produced 12.8 g/L lactate, 34.7 g/L ethanol, 6.9 g/L glycerol, 3.0 g/L acetate and 0.3 g/L pyruvate. After 47 hours at 42°C, the PDC- *Lh-D-LDH* strains CD558 and CD587 had consumed 80 g/L glucose and produced 75 g/L lactate, 2.5 g/L ethanol and 1.5 g/L acetate. In contrast, wild-type strain CD21 consumed 115.5 g/L glucose, and produced principally ethanol (39.7 g/L) and a small amount of lactate (1.0 g/L).

After 47 hours, the PDC+ *Lh-D-LDH* strain CD588 fermenting at 50°C had consumed 49.6 g/L glucose and produced 6.8 g/L lactate, 9.5 g/L ethanol, 4.5 g/L glycerol, and 1.8 g/L acetate. After 47 hours at 50°C, the PDC- *Lh-D-LDH* strains CD558 and CD587 had consumed 15 g/L glucose and produced 10 g/L lactate, 1.2-1.4 g/L pyruvate, and 1.6-1.7 g/L acetate. In contrast, wild-type strain CD21 consumed 70.5 g/L glucose and produced principally ethanol (13.6 g/L) and a small amount of lactate (0.5 g/L).

These results demonstrate that the D-Ldh+ transformants can produce D-lactic acid at temperatures as high as 50°C. The lactic acid produced by these strains is greater than 99% enantiomerically pure D-lactic acid, as determined by enzymatic analysis. Although the overall glucose consumption and lactate production rates decrease with increasing temperature, the yield of lactate from glucose remains between 88-100% for the PDC- *Lh-D-LDH* strains CD558 and CD587.

### Example 3H: Production of D-lactic acid in unbuffered complex media in microaerobic shake flask cultures of K. marxianus with a single copy of L. helveticus D-LDH integrated into the chromosome, strains

Wild-type *K. marxianus* strain CD21, the PDC+ *Lh-D-LDH* strain CD588, and the PDC- *Lh-*D*-LDH* strains CD558 and CD587 were were inoculated to an OD₆₀₀ of 0.1 from YPD agar plates and grown for 16 hours at 30°C with shaking at 250 rpm, in 250 mL baffled shake flasks containing 50 mL YPD supplemented with an additional 100 g/L glucose. After determining that residual glucose remained in each flask and that the cells were consequently in exponential growth phase, 4 g/L cell dry weight equivalents were harvested by centrifugation and resuspended in 250 mL baffled shake flasks containing 50 mL YPD supplemented with 40 g/L glucose, in duplicate. The cultures were placed at 37°C with 70 rpm. Samples were withdrawn at various time intervals and the cells were removed by filtration. Culture supernatants were analyzed for glucose, lactate, pyruvate, acetate, glycerol and ethanol by HPLC.

After 23 hours, the PDC+ *Lh-D-LDH* strain CD588 had consumed 58.9 g/L glucose, and produced 0.9 g/L lactate, 0.2 g/L pyruvate, 2.6 g/L glycerol, and 0.6 g/L acetate. The final culture pH was measured as 4.6. Ethanol was the main product from this fermentation, at 24-24.7 g/L. After 23 hours, PDC- *Lh-D-LDH* strains CD558 and CD587 had consumed 4.1 g/L glucose and produced between 2.5 g/L lactate, 1.0 g/L pyruvate, 0.8 g/L glycerol, and 0.4 g/L acetate. The final culture pH for the CD587 and CD558 strains ranged from 3.31-3.65. Lactate was the main product of the fermentation, at a level ranging from 1.2-3.3 g/L. Wild-type strain CD21 consumed 58.9 g/L glucose in 23 hours and produced mostly ethanol (12.8-20.4 g/L) and a small amount of lactate (1.2-1.3 g/L), with a final pH of 4.65.

These results show that the D-Ldh+ transformants produce D-lactic acid at pH values as low as 3.31. Enzymatic analysis of the lactic acid produced by these strains showed that the lactic acid in greater than 99% enantiomerically pure D-lactic acid.

### Example 31: D-lactic acid production from D-xylose in strains

Three engineered *K. marxianus* strains were analyzed for D-lactic acid production from D-xylose relative to CD21 (wild-type), including CD558 *(PDC1* null containing randomly integrated *L. helveticus D-LDH),* CD587 *(PDC1::Lh-D-LDH),* and CD588 (containing *L. helveticus D-LDH).* The following results demonstrate that the engineered strains can produce D-lactic acid from a non-glucose substrate.

Cells of strains CD21, CD558, CD587, and CD588 were grown on YPD + 20 g/L D-xylose agar plates and used to inoculate baffled shake flasks containing 100 mL of culture medium that contained 20g/L yeast extract, 10 g/L peptone, 17 g/L CaCO₃, and 50g/L D-xylose. The inoculated shake flasks were incubated at 35°C and shaken at 250 rpm. Samples were taken from the flasks and the OD₆₀₀ₙₘ measured in order to monitor culture growth and sugar utilization. After about 36 hours, the cells were collected by centrifugation, and cell dry weight (CDW) of each culture was determined. Into duplicate baffled shake flasks containing 20 g/L yeast extract, 10 g/L peptone, and 50g/L D-xylose was added 3.0 g/L CDW of cells from each strain. The flasks were incubated at 35°C with shaking at 70 rpm. Samples were taken at various time points for HPLC analysis and quantification of the medium components, including xylose, D-lactate, xylitol, and ethanol.

The main organic component produced by strains CD558 and CD587 was D-lactic acid, which reached an average highest titer of 13.7 g/kg. This results in a D-lactic acid yield from D-xylose of about 33%. The main organic component produced by strain CD21 is xylitol, which reached an average highest titer of 25.0 g/kg. This results in a xylitol yield from D-xylose of about 50%. The main organic component produced by strain CD588 was xylitol, which reached an average highest titer of 19.6 g/kg. This results in an average xylitol yield from D-zylose of about 44%. HPLC analysis did not detect any glycerol, citric, pyruvate, succinic, or acetate production in any of the strains.

### Example 4: Plamids for expression of the B. megaterium D-LDH and for targeted integration of the transformed DNA into the PDC1 locus

Plasmids comprising a *B. megaterium* D-LDH gene for targeted integration into the *C. sonorensis PDC1* gene locus are prepared as follows:
Plasmid pMI257 (Candida application) is linearized with *Nco*I and the 5' overhangs were partially filled in with DNA polymerase I, Klenow fragment, and a mixture of dATP, dCTP, and dTTP, omitting dGTP from the reaction. This is followed by removal of the single stranded extensions by treatment with mung bean nuclease. The DNA is then digested with *Bam*HI and the 9200 bp fragment isolated from a 0.8% agarose gel after electrophoretic separation. Plasmid pVR47 (Ex. 1J) containing *B*. *megaterium* D-LDH is generated from *B. megaterium* genomic DNA, and is shown in FIG. 12. The 1023 bp fragment containing the *B. megaterium* LDH is excised from pVR47 by *Xba*I and digestion followed by fill-in of the 5' overhangs by DNA polymerase I, Klenow fragment and each of the 4 dNTPs, and digestion by *Bam*HI*.* The 9200 bp *Nco*I (blunt)-*Bam*HI fragment from pMI257 and the 976 bp *Xba*I(blunt)-*Bam*HI fragment from pVR47 are ligated and the resulting plasmid is designated as pMIXXX, shown in FIG. 18. pMI1000 contains, in order, the *C. sonorensis PDC1* promoter, the C. *sonorensis PGK1* promoter operatively linked to the *S. cerevisiae MEL5* gene, the *C. sonorensis PGK1* promoter operatively linked to the B. *megaterium* D-LDH and the *C. sonorensis PDC1* terminator. PMI1000 is digested with *Not*I to excise the 7300 bp fragment that consists of the MEL5 and LDH expression cassettes flanked by the *PDC1* 5' and 3' regions. This 7500 bp fragment is used to transform *C. sonorensis* (Candida filing) and the transformants are screened on YPD plates supplemented with X-α-gal at a concentration of 40 µg/mL. The transformants are grown on YPD agar plates supplemented with X-α-gal (40 µg/mL).

## Claims

1. A recombinant yeast cell of a species that does not naturally accumulate pyruvate, comprising at least one exogenous D-lactate dehydrogenase gene integrated into its genome, wherein the D-lactate dehydrogenase gene is operatively linked to functional promoter and terminator sequences.

2. The recombinant yeast cell of claim 1, which is of the genera Candida or Kluyveromyces.

3. The recombinant yeast cell of claim 2, wherein the cell is of the species *C. sonorensis* or *K*. *marxianus.*

4. The recombinant yeast cell of claim 1, wherein the promoter sequence is at least 90% homologous to a promoter that is native to the yeast species.

5. The recombinant yeast cell of claim 4, wherein the terminator sequence is at least 90% homologous to a terminator that is native to the yeast species.

6. The recombinant yeast cell of claim 4, wherein the yeast species contains a native PDC gene having a native PDC promoter, and the promoter sequence is at least 90% homologous to the native PDC promoter.

7. The recombinant yeast cell of claim 6, wherein the native PDC gene has a native PDC terminator, and the terminator sequence is at least 90% homologous to the native PDC terminator.

8. The recombinant yeast cell of claim 7, wherein the native PDC gene is deleted.

9. The recombinant yeast cell of claim 1, wherein the yeast species contains a native PDC gene, and the native PDC gene is deleted.

10. The recombinant yeast cell of claim 1, wherein the nucleotide sequence encodes a D-lactate dehydrogenase protein from *Lactobacillus helueticus, Lactobacillus johnsonii*, *Lactobacillus bulgaricus, Lactobacillus delbrueckii,* lactobacillus *plantarum and Lactobacillus pentosus.*

11. The recombinant yeast cell of claim 10, wherein the promoter is from a yeast species from the genera *Kluyveromyces* or *Saccharomyces*.

12. The recombinant yeast cell of claim 11, wherein the promoter is from *Kluyveromyces marxianus.*

13. The recombinant yeast cell of claim 11, wherein the promoter is from *Saccharomyces cerevisiae.*

14. The recombinant yeast cell of claim 1 which exhibits reduced PDC activity.

15. The recombinant yeast cell of claim 8 wherein the D-LDH gene is integrated at the locus of the deleted PDC gene.

16. A method for fermenting a carbohydrate to lactic acid comprising culturing the cell of claim 1 under fermentation conditions in a medium containing a carbohydrate that is fermentable by the cell.

17. A method for fermenting a carbohydrate to lactic acid comprising culturing the cell of claim 8 under fermentation conditions in a medium containing a carbohydrate that is fermentable by the cell.

18. A method for fermenting a carbohydrate to lactic acid comprising culturing the cell of claim 9 under fermentation conditions in a medium containing a carbohydrate that is fermentable by the cell.

19. A method for fermenting a carbohydrate to lactic acid comprising culturing the cell of claim 14 under fermentation conditions in a medium containing a carbohydrate that is fermentable by the cell.

20. A method according to the method of claims 16, 17, 18, or19, further comprising the step of converting at least a portion of the lactic acid to lactide.

21. The method of claim 20, further comprising the step of polymerizing the lactide to form a polylactide polymer or copolymer.

22. A method for producing lactic acid comprising culturing the cell of claim 1 under fermentation conditions in a medium containing a sugar that is fermentable by the cell.

23. A method for producing lactic acid comprising culturing the cell of claim 8 under fermentation conditions in a medium containing a sugar that is fermentable by the cell.

24. A method for producing lactic acid comprising culturing the cell of claim 9 under fermentation conditions in a medium containing a sugar that is fermentable by the cell.

25. A method for producing lactic acid comprising culturing the cell of claim 14 under fermentation conditions in a medium containing a sugar that is fermentable by the cell.

26. A method according to claims 22, 23, 24, or 25, further comprising the step of converting at least a portion of the lactic acid to lactide

27. The method of claim 26, further comprising the step of polymerizing the lactide to form a polylactide polymer or copolymer.

## Patentansprüche

1. Rekombinante Hefezelle einer Spezies, die natürlicherweise kein Pyruvat ansammelt, umfassend wenigstens ein exogenes D-Lactatdehydrogenasegen, das in ihr Genom integriert ist, wobei das D-Lactatdehydrogenasegen funktionsfähig mit funktionalen Promotor- und Terminatorsequenzen verbunden ist.

2. Rekombinante Hefezelle nach Anspruch 1, die von der Gattung Candida oder Kluyveromyces ist.

3. Rekombinante Hefezelle nach Anspruch 2, wobei die Zelle von der Spezies *C. sonorensis* oder *K. marxianus* ist.

4. Rekombinante Hefezelle nach Anspruch 1, wobei die Promotorsequenz wenigstens 90% homolog zu einem Promoter ist, welcher der Hefespezies nativ ist.

5. Rekombinante Hefezelle nach Anspruch 4, wobei die Terminatorsequenz wenigstens 90% homolog zu einem Terminator ist, welcher der Hefespezies nativ ist.

6. Rekombinante Hefezelle nach Anspruch 4, wobei die Hefespezies ein natives PCD-Gen mit einem nativen PDC-Promotor enthält und die Promotorsequenz wenigstens 90% homolog zu dem nativen PDC-Promotor ist.

7. Rekombinante Hefezelle nach Anspruch 6, wobei das native PDC-Gen einen nativen PDC-Terminator hat und die Terminatorsequenz wenigstens 90% homolog zu dem nativen PDC-Terminator ist.

8. Rekombinante Hefezelle nach Anspruch 7, wobei das native PDC-Gen deletiert ist.

9. Rekombinante Hefezelle nach Anspruch 1, wobei die Hefespezies ein natives PDC-Gen enthalten und das native PDC-Gen deletiert ist.

10. Rekombinante Hefezelle nach Anspruch 1, wobei die D-Lactatdehydrogenaseprotein aus *Lactobacillus helveticus, Lactobacillus johnsonii, Lactobacillus bulgaricus, Lactobacillus delbrueckii, Lactobacillus plantarum* und *Lactobacillus pentosus* codiert.

11. Rekombinante Hefezelle nach Anspruch 10, wobei der Promotor aus einer Hefespezies aus der Gattung *Kluyveromyces* oder *Saccaromyces* ist.

12. Rekombinante Hefezelle nach Anspruch 11, wobei der Promotor aus *Kluyveromyces marxianus* ist.

13. Rekombinante Hefezelle nach Anspruch 11, wobei der Promotor aus *Saccaromyces cerevisiae* ist.

14. Rekombinante Hefezelle nach Anspruch 1, welche verminderte PDC-Aktivität zeigt.

15. Rekombinante Hefezelle nach Anspruch 8, wobei das D-LDH-Gen an dem Locus des deletierten PDC-Gens integriert ist.

16. Verfahren zur Fermentierung eines Kohlehydrats zu Milchsäure, umfassend das Kultivieren der Zelle nach Anspruch 1 unter Fermentationsbedingungen in einem Medium, das ein Kohlehydrat enthält, welches durch die Zelle fermentierbar ist.

17. Verfahren zur Fermentierung eines Kohlehydrats zu Milchsäure, umfassend das Kultivieren der Zelle von Anspruch 8 unter Fermentationsbedingungen in einem Medium, das ein Kohlehydrat enthält, welches durch die Zelle fermentierbar ist.

18. Verfahren zur Fermentierung eines Kohlehydrats zu Milchsäure, umfassend das Kultivieren der Zelle von Anspruch 9 unter Fermentationsbedingungen in einem Medium, das ein Kohlehydrat enthält, welches durch die Zelle fermentierbar ist.

19. Verfahren zur Fermentierung eines Kohlehydrats zu Milchsäure, umfassend das Kultivieren der Zelle von Anspruch 14 unter Fermentationsbedingungen in einem Medium, das ein Kohlehydrat enthält, welches durch die Zelle fermentierbar ist.

20. Verfahren nach einem der Ansprüche 16, 17, 18 oder 19, weiterhin umfassend den Schritt des Umwandelns wenigstens eines Teils der Milchsäure zu Lactid.

21. Verfahren nach einem der Ansprüche 20, weiterhin umfassend den Schritt des Polymerisierens des Lactids, um ein Polylactidpolymer oder -copolymer zu bilden.

22. Verfahren zum Erzeugen von Milchsäure, umfassend das Kultivieren der Zelle von Anspruch 1 unter Fermentationsbedingungen in einem Medium, das einen Zucker enthält, der durch die Zelle fermentierbar ist.

23. Verfahren zum Erzeugen von Milchsäure, umfassend das Kultivieren der Zelle von Anspruch 8 unter Fermentationsbedingungen in einem Medium, das einen Zucker enthält, der durch die Zelle fermentierbar ist.

24. Verfahren zum Erzeugen von Milchsäure, umfassend das Kultivieren der Zelle von Anspruch 9 unter Fermentationsbedingungen in einem Medium, das einen Zucker enthält, der durch die Zelle fermentierbar ist.

25. Verfahren zum Erzeugen von Milchsäure, umfassend das Kultivieren der Zelle von Anspruch 14 unter Fermentationsbedingungen in einem Medium, das einen Zucker enthält, der durch die Zelle fermentierbar ist.

26. Verfahren nach einem der Ansprüche 22, 23, 24 oder 25, weiterhin umfassend den Schritt des Umwandelns wenigstens eines Teils der Milchsäure in Lactid.

27. Verfahren nach Anspruch 26, weiterhin umfassend den Schritt des Polymerisierens des Lactids, um ein Polylactidpolymer oder -copolymer zu bilden.

## Revendications

1. Cellule de levure recombinante d'une espèce qui n'accumule pas naturellement du pyruvate, comprenant au moins un gène de D-lactate déshydrogénase exogène intégré dans son génome, où le gène de D-lactate déshydrogénase est lié de manière fonctionnelle à des séquences promotrice et terminatrice fonctionnelles.

2. Cellule de levure recombinante selon la revendication 1, qui est du genre Candida ou Kluyveromyces.

3. Cellule de levure recombinante selon la revendication 2, où la cellule est de l'espèce *C. sonorensis* ou *K. marxianus.*

4. Cellule de levure recombinante selon la revendication 1, où la séquence promotrice est homologue à raison d'au moins 90 % d'un promoteur qui est natif de l'espèce de levure.

5. Cellule de levure recombinante selon la revendication 4, où la séquence terminatrice est homologue à raison d'au moins 90 % d'un terminateur qui est natif de l'espèce de levure.

6. Cellule de levure recombinante selon la revendication 4, où l'espèce de levure contient un gène de PDC natif ayant un promoteur de PDC natif, et la séquence promotrice est homologue à raison d'au moins 90 % du promoteur de PDC natif.

7. Cellule de levure recombinante selon la revendication 6, où le gène de PDC natif a un terminateur de PDC natif, et la séquence terminatrice est homologue à raison d'au moins 90 % du terminateur de PDC natif.

8. Cellule de levure recombinante selon la revendication 7, où le gène de PDC natif est délété.

9. Cellule de levure recombinante selon la revendication 1, où l'espèce de levure contient un gène de PDC natif, et le gène de PDC natif est délété.

10. Cellule de levure recombinante selon la revendication 1, où la séquence nucléotidique code une protéine D-lactate de *Lactobacillus helveticus, Lactobacillus johnsonii, Lactobacillus bulgaricus, Lactobacillus delbrueckii, Lactobacillus plantarum* et *Lactobacillus pentosus.*

11. Cellule de levure recombinante selon la revendication 10, où le promoteur provient d'une espèce de levure du genre *Kluyveromyces* ou *Sacceharomyces*.

12. Cellule de levure recombinante selon la revendication 11, où le promoteur provient de *Kluyveromyces marxianus*.

13. Cellule de levure recombinante selon la revendication 11, où le promoteur provient de *Saccharomyces cerevisiae.*

14. Cellule de levure recombinante selon la revendication 1, qui présente une activité PDC réduite.

15. Cellule de levure recombinante selon la revendication 8, où le gène de D-LDH est intégré au locus du gène de PDC délété.

16. Procédé pour fermenter un glucide en acide lactique comprenant la culture de la cellule selon la revendication 1 dans des conditions de fermentation dans un milieu contenant un glucide qui est fermentable par la cellule.

17. Procédé pour fermenter un glucide en un acide lactique comprenant la culture de la cellule selon la revendication 8 dans des conditions de fermentation dans un milieu contenant un glucide qui est fermentable par la cellule.

18. Procédé pour fermenter un glucide en acide lactique comprenant la culture de la cellule selon la revendication 9 dans des conditions de fermentation dans un milieu contenant un glucide qui est fermentable par la cellule.

19. Procédé pour fermenter un glucide en acide lactique comprenant la culture de la cellule selon la revendication 14 dans des conditions de fermentation dans un milieu contenant un glucide qui est fermentable par la cellule.

20. Procédé selon le procédé selon les revendications 16, 17, 18 ou 19 comprenant en outre l'étape de conversion d'au moins une partie de l'acide lactique en lactide.

21. Procédé selon la revendication 20 en outre l'étape de polymérisation du lactide pour former un polymère ou copolymère polylactide.

22. Procédé pour produire de l'acide lactique comprenant la culture de la cellule selon la revendication 1 dans des de fermentation dans un milieu contenant un sucre qui est fermentable par la cellule.

23. Procédé pour produire de l'acide lactique comprenant la culture de la cellule selon la revendication 8 dans des conditions de fermentation dans un milieu contenant un sucre qui est fermentable par la cellule.

24. Procédé pour produire de l'acide lactique comprenant la culture de la cellule selon la revendication 9 dans des conditions de fermentation dans un milieu contenant un sucre qui est fermentable par la cellule.

25. Procédé pour produire de l'acide lactique comprenant la culture de la cellule selon la revendication 14 dans des conditions de fermentation dans un milieu contenant un sucre qui est fermentable par la cellule.

26. Procédé selon les revendications 22, 23, 24 ou 25 comprenant en outre l'étape de conversion d'au moins une partie de l'acide lactique en lactide.

27. Procédé selon la revendication 26 comprenant en outre l'étape de polymérisation du lactide pour former un polymère ou copolymère polylactide.
